# EUROPÄISCHE PATENTSCHRIFT

(11) **EP 3 355 830 B1**
(45) Veröffentlichungstag und Bekanntmachung des Hinweises auf die Patenterteilung: **15.07.2020**
(21) Anmeldenummer: 16788012.9
(22) Anmeldetag: 27.09.2016
(51) Int. Cl.: B22F 3/105, B22F 7/08, A61F 2/30, A61F 2/36, B33Y 80/00, B33Y 10/00, B33Y 40/00, G01D 11/24, H01L 23/04, A61F 2/46, B22F 5/10, A61F 2/48

(54) **VERBUNDKÖRPER MIT MINDESTENS EINER FUNKTIONSKOMPONENTE UND EIN VERFAHREN ZUR HERSTELLUNG DES VERBUNDKÖRPERS**
COMPOSITE BODY HAVING AT LEAST ONE FUNCTIONAL COMPONENT, AND A METHOD FOR PRODUCING SAID COMPOSITE BODY
CORPS COMPOSITE COMPRENANT AU MOINS UN ÉLÉMENT FONCTIONNEL ET PROCÉDÉ POUR RÉALISER LE CORPS COMPOSITE

(30) Priorität: 28.09.2015 DE 102015116409
(43) Veröffentlichungstag der Anmeldung: 08.08.2018
(73) Patentinhaber: Fraunhofer-Gesellschaft zur Förderung der angewandten Forschung e.V., 80686 München (DE)
(72) Erfinder: LAUSCH, Holger, 04299 Leipzig (DE); HERRMANN, Mathias, 01640 Coswig (DE); GRONDE, Bernd, 07629 Schleifreisen (DE); TÖPPEL, Thomas, 01877 Bischofswerda (DE); PETTERS, Romy, 01705 Freital (DE); ROTSCH, Christian, 01468 Moritzburg (DE)
(74) Vertreter: Oehmke, Volker
(86) Internationale Anmeldenummer: PCT/DE2016/100450
(87) Internationale Veröffentlichungsnummer: WO 2017/054799

(56) Entgegenhaltungen:
- WO-A2-2014/209994
- US-A- 5 996 219
- US-A1- 2007 177 362
- US-A1- 2015 144 386

## Beschreibung

Die Erfindung betrifft einen Verbundkörper, bei dem mindestens eine Funktionskomponente in einem Formkörper integriert ist, und ein Verfahren zu seiner Herstellung. Der Formkörper kann dabei insbesondere ein Implantat, eine Prothese, ein industrielles Bauteil oder eine multifunktional nutzbare Sensorplattform zum Material-, Bauteil- und / oder Tragwerkmonitoring sein.

Additive (generative) Fertigungsverfahren haben in den letzten drei Jahrzehnten enorme Fortschritte gemacht, was die Werkstoffvielfalt, Produktivität, Genauigkeit und Materialeigenschaften betrifft. Aus diesem Grund werden generative Verfahren zunehmend auch für die Serienfertigung komplexer Bauteile eingesetzt. Besonders die pulverbettbasierten Verfahren, wie das Laserstrahlschmelzen für metallische Werkstoffe, zeigen die größten Potentiale, um geometrisch komplexe Werkstücke und Werkzeuge in serienidentischen Werkstoffen mit serienidentischen Eigenschaften konventioneller Fertigungsmethoden, wie z. B. Fräsen oder Gießen, herstellen zu können. Alternativ ist aus der US 2007/0177362 A1 ein Festkörper-Additives-Fertigungsverfahren bekannt, wobei ein schichtweises Verschweißen von Metallblechen oder -folien durch Reibung (Reibschweißen) erfolgt. Die geometrische Komplexität der herstellbaren Bauteile ist hierbei gegenüber pulverbettbasierten Verfahren, wie dem Laserstrahlschmelzen, deutlich eingeschränkt.

Ein Formkörper wird additiv durch schichtweises ortsselektives Verfestigen mit lokal definiertem Energieeintrag in pulverförmige, aufschmelzbare Werkstoffe hergestellt. Der Energieeintrag erfolgt dabei durch einen Energiestrahl, insbesondere einen Laserstrahl oder Elektronenstrahl. Bekannte Verfahrensbezeichnungen hierfür sind "Selective Laser Melting (SLM)", "Laser Melting", "Laserschmelzen", "Laserforming", "LaserCusing" oder "Direktes Metall-Laser-Sintern (DMLS)", "Electron Beam Melting (EBM)". Im Weiteren wird dieses Verfahren als Strahlschmelzen zusammengefasst.

In Strahlschmelzanlagen können dreidimensionale Körper nach dem Schichtaufbauprinzip hergestellt werden, wobei durch einen Energieeintrag, z. B. per Laserstrahl, einzelne Pulverschichten (Layer) nacheinander ortsselektiv aufgeschmolzen werden und zu einem festen Formkörper erstarren. Dabei kann über eine Schiebevorrichtung das Werkstoffpulver, das z. B. über eine Pulvervorratskammer bereitgestellt wird, über eine Bauplattform in einer Prozesskammer verteilt werden, indem die Schiebevorrichtung horizontal verfahren wird, um so eine typischerweise 20 µm bis 150 µm dicke Schicht an Werkstoffpulver programmierbar definiert aufzutragen. Im Anschluss scannen ein oder mehrere Laserstrahlen, z. B. mittels Faser- oder Diodenlasern, die zu schmelzenden Bereiche der aufgetragenen Pulverschicht selektiv. Nach Abschluss des lokalen Aufschmelzens der Schicht senkt sich die Bauplattform der Prozesskammer um die gewählte Schichtdicke ab, damit eine weitere Schicht aufgetragen werden kann, so dass das Werkstück schichtweise hergestellt werden kann.

Eine derartige Vorrichtung und ein Verfahren zur additiven Herstellung eines Werkstücks/Formkörpers durch Laserstrahlschmelzen eines pulverförmigen Werkstoffes ist aus WO 98/24574 A1 bekannt.

Bei einer solchen zwar sehr flexiblen Möglichkeit zur Herstellung derartiger Formkörper treten durch das Strahlschmelzen üblicherweise sehr hohe Temperaturen auf.

Sollten in oder an den Formkörper weitere Elemente integriert werden, ist eine Herstellung des Verbundes in einem Fertigungsprozess kaum möglich.

Es ist daher Aufgabe der Erfindung, Möglichkeiten zur Gestaltung und Herstellung eines Verbundkörpers anzugeben, bei dem mindestens eine Funktionskomponente in einen Formkörper, der generativ aus einem metallischen Pulver durch Strahlschmelzen hergestellt wird, integriert werden kann, ohne dass es durch die erhöhten Temperaturen zu einer Beeinträchtigung der Funktionalität der jeweiligen Funktionskomponente kommt.

Die Aufgabe wird durch einen Verbundkörper mit mindestens einer Funktionskomponente und einem Formkörper, dadurch gelöst, dass die mindestens eine Funktionskomponente mit einer mindestens ein Konturelement aufweisenden metallischen oder keramischen Trägerstruktur in Verbindung stehend angeordnet ist und die mindestens eine Funktionskomponente bis auf die Konturelemente von mindestens einer thermisch isolierenden keramischen Schicht umschlossen und darüber mindestens bereichsweise mit thermisch leitfähigem Metall oder Metalloxid beschichtet ist, so dass keine stoffschlüssige Verbindung zwischen Funktionskomponente und Formkörper besteht. Der durch Strahlschmelzen eines Metallpulvers generativ hergestellte Formkörper umschließt die mindestens eine Funktionskomponente und die Trägerstruktur mit dem mindestens einen Konturelement dabei ganz. Von dem mindestens einen Konturelement weist wenigstens eins eine stoffschlüssige Verbindung zum Formkörper auf.

Der Verbundkörper ist ebenfalls dadurch charakterisiert, dass die Architektur und der Aufbau der Funktionskomponente zum Formkörper so gestaltet sind, dass einerseits ein Energiefluss und eine Übertragung von außerhalb des Formkörpers zur Funktionskomponente möglich ist und andererseits zwischen der Funktionskomponente und dem Formkörper ein Energiefluss und Energieübertragung generell und insbesondere nach der stofflichen Integration mittels Strahlschmelzen in- und exklusive thermischen und/oder mechanischen Nachbehandlungsverfahren, insbesondere Fügeund Verdichtungsverfahren, funktionserhaltend möglich sind. Die Nachbehandlungsverfahren gewährleisten einerseits eine stoffliche, chemische und korrosionstechnische Separation der Funktionskomponente gegenüber dem den Formkörper umgebenden Medium oder Einflüssen sowie andererseits die Diffusion von chemischen und stofflichen Bestandteilen der Funktionskomponente durch den Formkörper hindurch an die den Formkörper umgebende Umwelt. Dies muss auch bei einer mechanischen beziehungsweise traumatischen Deformation des Formkörpers gewährleistet sein. Daraus ergibt sich die Notwendigkeit der thermischen und/oder mechanischen Nachbehandlung des Formkörpers oder einer angepassten Konstruktionsweise/Architektur des Formkörpers. Im Ergebnis ist die Funktionskomponente hermetisch/dicht in den Formkörper eingeschlossen, sodass für die Funktionskomponente ein chemischer und mechanischer Schutz (z. B. Druck- oder Verschleißschutz) gegenüber den Umwelteinflüssen außerhalb des Verbundkörpers sichergestellt wird. Zudem stellt der Formkörper um die Funktionskomponente auch einen thermischen Schutz der Funktionskomponenten vor äußeren Umwelteinflüssen dar. Umgekehrt kann der Formkörper auch als chemische und/oder mechanische und/oder thermische Barriere wirken, um die den Verbundkörper umgebende Umwelt vor chemischen und/oder mechanischen und/oder thermischen Einflüssen der Funktionskomponente zu schützen.

Neben der üblichen form- und/oder stoffschlüssigen Integration einer Funktionskomponente in einen Formkörper sind Ausführungen möglich, die vorrangig eine Wechselwirkung der beiden Elemente untereinander beziehungsweise mit der Umwelt zum Ziel haben oder die den Stoff- und/oder Formschluss der Funktionskomponente, z. B. eines Piezoaktors, oder die Gewährleistung eines Gegenlagers oder einer Spannvorrichtung für diesen zum Ziel haben, ist eine monolithische form- und stoffschlüssige Einbettung nicht notwendig, sondern eher einer Schwingungsübertragung hinderlich. Die Funktionskomponente kann im Formkörper so integriert werden, dass diese einerseits in mindestens einer Dimension gegenüber dem Formkörper beweglich ist sowie andererseits nicht ausgefüllte Volumina (z. B. Luft, Schutzgas oder unaufgeschmolzenes Pulvermaterial) aufweist.

Der Integrationsschluss muss so gestaltet sein, dass zwischen der Funktionskomponente und dem Formkörper eine Gradientenübertragung oder ein Gradientenausgleich zur Bildung eines physikalischen Gleichgewichtes in Gestalt eines Energietransports möglich ist. So kann das Einkoppeln von mechanischer Energie in Gestalt von Schwingungen und Eigenspannungsunterschieden, die z. B. erst an der Oberfläche des Formkörpers bzw. eines definierten Areals von Körperschall zu Raumschall gewandelt werden, erfolgen. Durch Gewährleistung mindestens eines geometrischen Freiheitsgrades der Funktionskomponente und eines Kraftübertragungskorridors zum Formkörper, der z. B. mittels einer Nut-Feder-Verbindung über Strahlschmelzen form-, kraft- und stoffschlüssig verbunden wird, wird eine definierte Gradientenübertragung im oben genannten Sinne möglich. Im Falle einer thermischen Energieeinkopplung ist ebenfalls eine monolithische form- und stoffschlüssige Einbettung nicht an sich notwendig. Die z. B. in einer Funktionskomponente durch Wandlung entstandene thermische Energie kann über sogenannte thermisch isolierte Wärmeleiter (Thermotunnel) mit umgebenden Wärmeisolierungen oder Luft bzw. Schutzgas (Thermoskanneneffekt) oder nicht aufgeschmolzenem Pulver im Formkörper bis zu anderen Funktionskomponenten im Formkörper und/oder an der Oberfläche des Formkörpers im Sinne einer thermischen Gleichgewichtbildung so weitergeleitet werden, dass keine oder nur eine geringe Erwärmung des Gesamtformkörpers stattfindet. Ebenso gilt das für die Einkopplung von elektrischer/elektromagnetischer Energie inkl. Remanenzen und translatorischen Kräfte von der wandelnden Funktionskomponente an topische Funktionsbereiche innerhalb und/oder auf der Oberfläche des Formkörpers durch elektrisch isolierte, z. B. keramisch ummantelte, elektrische Flussstrecken. In allen genannten Fällen wird über thermisch, elektrisch oder mechanisch ummantelte, über Freiheitsgrade eingeschränkte Überträger ein energetisches Gleichgewicht zwischen der Funktionskomponente und topischen Arealen in oder an der Oberfläche des Formkörpers im Sinne eines Energiegleichgewichts hergestellt. Die anderen Funktionskomponenten im Formkörper bzw. an der Oberfläche des Formkörpers und/oder auch topische Areale des Formkörpers selbst stellen eine Art Verbraucher oder Last gegenüber einer primären energiewandelnden und/oder signalgenerierenden Funktionskomponente dar. In jedem Falle beeinflusst die primäre Funktionskomponente den Formkörper bzw. andere sekundäre Funktionskomponenten und die den Formkörper umgebende Umwelt. Dies gilt auch reversiv, sodass eine aktorische Funktionskomponente zur sensorischen Funktionskomponente werden kann.

Die Aufgabe wird ferner durch ein Verfahren zur Herstellung eines Verbundkörpers, dadurch gelöst, dass zunächst mindestens eine Funktionskomponente mit einer metallischen oder keramischen Trägerstruktur verbunden wird, anschließend die mindestens eine Funktionskomponente bis auf die Konturelemente umschließend mit einer thermisch isolierenden keramischen Schicht versehen wird, anschließend mindestens bereichsweise darüber eine thermisch leitfähige Schicht aus einem Metall oder Metalloxid aufgetragen wird, anschließend ein Teilkörper mit mindestens einer Strahlschmelzzone und Aussparung zur Aufnahme der bisher hergestellten Einheit durch Strahlschmelzen eines Metallpulvers generativ und schichtweise hergestellt wird, anschließend die bisher hergestellte Einheit in den Teilkörper form- und/oder kraftschlüssig in die Aussparung eingesetzt wird, wobei die Trägerstruktur mittelbar oder unmittelbar über Konturelemente und/oder Noppen mit der Strahlschmelzzone stoffschlüssig mit dem Teilkörper verbunden wird, und abschließend ebenfalls durch Strahlschmelzen eines Metallpulvers generativ und schichtweise der Teilkörper mit mindestens einem weiteren Teilkörper überbaut wird, so dass ein monolithischer Formkörper entsteht.

Der Energiestrahl sollte dabei beim Strahlschmelzen so betrieben und mit seinem Brennfleck so bewegt werden, dass eine für die jeweilige Funktionskomponente spezifische Maximaltemperatur während der Herstellung des Formkörpers nicht überschritten wird. Die Energiedichte im Brennfleck des Energiestrahls, die Vorschubgeschwindigkeit des Brennflecks, der Abstand von Schmelzspuren, die jeweilige Schichtdicke der Pulverschichten und/oder das Bestrahlungsmuster können so beeinflusst werden, dass die spezifische Maximaltemperatur an der Funktionskomponente nicht erreicht wird. Vorteilhaft ist es, wenn in den der O140-12469-EP Funktionskomponente angrenzenden Bereichen mit einem um mindestens 10 %, bevorzugt um mindestens 20 % reduzierten Energieeintrag gearbeitet wird.

Der Strahlschmelzprozess wird vorteilhaft so durchgeführt, dass z. B. die mechanischen Schwingungs- oder Spannungsänderungen über entsprechende geometrische Freiheitsgrade von der energieaufnehmenden und -wandelnden Funktionskomponente zum Formkörper gewährleistet werden können.

Ein Hohlraum kann zugleich Integrationsraum innerhalb des Formkörpers mit entsprechenden stofflichen Verbindungsstellen (Schmelzzonen bzw. Integrationszonen) sein. Die Trägerstruktur ist dementsprechend mit einem oder mehreren Konturelementen ausgestattet.

Der bewusste Verzicht auf eine formschlüssige monolithische Ummantelung der Funktionskomponente mit dem umschließenden Formkörper schafft somit ungefüllte beziehungsweise nicht dicht gefüllte Hohl- oder Zwischenräume, die einen zusätzlichen thermoisolierenden Effekt erreichbar machen. Über die Wahl der Konstruktion und Herstellungsschritte am Formkörper, wie z. B. bei der Wahl einer gewölbebildenden Ummantelung, kann man stabile formschlüssige Verbindungsbereiche und gleichzeitig thermoisolierende Bereiche ausbilden. Die Anordnungskonstruktion zwischen der integrierenden Schmelzzone am Formkörper sowie der integrierenden Schmelzzone an der Trägerstruktur der Funktionskomponente kann als Verbindungsschnittstelle so gestaltet werden, dass der thermische Energieeintrag im Bereich der Verbindungsschnittstelle von der thermisch empfindlicheren Funktionskomponente weg, hin zur stärker thermisch leitenden Verbindungsstelle des Formkörpers geleitet wird, indem an der Verbindungsschnittstelle der Werkstoffanteil des Formkörpers größer ist als im Bereich der Funktionskomponente. Dieser Effekt kann auch erhöht werden, wenn die geometrische Gestalt der Trägerstruktur im Bereich der Verbindungsschnittstelle zum Formkörper, beispielsweise an der Oberfläche, einen geringeren Wärmeleitungs- und/oder -speicherungskoeffizienten hat, als im Inneren der Trägerstruktur, also die Trägerstruktur mit einem entsprechenden Gradienten behaftet ist. Wenn die Trägerstruktur ebenfalls separat durch Strahlschmelzen hergestellt wird, so kann das Gefüge im Bereich der künftigen Verbindungsschnittstelle derart gestaltet werden, dass ein Form-, Kraft- und Stoffschluss möglich sind. Beim Strahlschmelzen sollten die Gefügestrukturen (Kristallform, Materialtyp, Legierung) adäquat auf den beiden Seiten der Strahlschmelzzone, d. h. im Bereich des Konturelements der Funktionskomponente und dem Formkörper, gleich sein. Dabei kann der Verbindungsschnittstellenbereich der Trägerstruktur eine noch geringere Wärmeleitfähigkeit aufweisen als der übrige Teil der Trägerstruktur der Funktionskomponente, der die thermisch empfindlichen Bauteile der Funktionskomponente aufnimmt. Dies kann auch erreicht werden, indem wie oben beschrieben die Trägerstruktur z. B. aus Keramik besteht und nur der Schnittstellenbereich metallisch beschichtet ist, was z. B. über thermisches Spritzen möglich ist.

Diese konstruktiven Aspekte bedingen auch Anpassungen im Herstellungsablauf im Strahlschmelzprozess. Die Qualität des form-, kraft- und stoffschlüssigen Verbundes im Verbindungsbereich hängt beim Strahlschmelzen wesentlich von der Prozessführung ab. Es sollte ein gleichmäßiger Pulverauftrag der aufzubauenden Schichten an der Verbindungsschnittstelle, aber auch an den ungefüllten beziehungsweise nicht dicht gefüllten Hohl- oder Zwischenräumen zur Funktionskomponente eingehalten werden. Sollte ein solches Areal (Volumen) nicht mit Pulver gefüllt werden oder mittels eines Manipulators durch eine thermisch isolierende Pulver- oder Kugelmischung (Granulat, Partikel etc.) befüllt werden, um beispielsweise eine bessere thermische Isolation zu erhalten beziehungsweise die Funktionskomponente vor dem Erreichen einer maximal zulässigen Temperatur, z. B. dem Schmelzpunkt, zu schützen, kann im Bereich, in dem kein Aufschmelzen des Pulvers bzw. der offenliegenden Funktionskomponentenoberfläche erwünscht ist, der Volumenenergieeintrag lokal definiert reduziert werden. Der Volumenenergieeintrag sollte in diesem Bereich aber nicht vollständig reduziert werden, damit keine Spannungsgradienten zwischen aufgeschmolzenen und unaufgeschmolzenen Bereichen des Metallpulvers während des Schmelzprozesses entstehen. Die Senkung des Volumenenergieeintrages findet seine Grenze immer an dem Funktionserhalt der Schnittstelle (Form-, Kraft- und Stoffschluss) und im statischen Erhalt der Funktion des Formkörpers sowie der eingebetteten Funktionskomponente (d. h. die Senkung des Volumenenergieeintrags hängt dadurch auch von den eingesetzten Wandstärken, vom Werkstoff, Design des Formkörpers ab). Vorteilhaft ist eine lokal definierte Prozesssteuerung des Volumenenergieeintrages, z. B. der Laserleistung, Vorschubgeschwindigkeit, dem Abstand der Schweißspuren, Bestrahlungsmuster und/oder der Schichtdicke, die einerseits ein lokal definiertes Aufschmelzen des Pulvers und andererseits eine lokal definierte spannungsreduzierende Wärmebehandlung des das Schmelzbad umgebenden Bereichs an der Funktionskomponentenoberfläche ermöglicht. Dies kann in der Regel über die Anlagen- und Prozesssteuerung gezielt erreicht werden. Die lokal definierte Prozesssteuerung im oben genannten Sinne sollte nicht nur zweidimensional in der jeweils aktuell aufzuschmelzenden Pulverschicht sinnvoll sein, sondern darüber hinaus auch über die Variation des Volumenenergieeintrags in den vorhergehenden und darauffolgenden Pulverschichten definiert verändert werden können. Diese spannungsreduzierende Wärmehandlung beinhaltet beispielsweise eine Prozesssteuerung, wobei das aufgetragene Metallpulver und/oder die darunter liegenden bereits geschmolzenen Schichten vor dem Strahlschmelzen der neu aufgetragenen Metallpulverschicht vorgewärmt werden. Dies ist besonders wichtig, wenn die bereits geschmolzenen Schichten z. B. nach innen offene Rahmen darstellen und der Strahlschmelzprozess diagonal über die Rahmenteile erfolgt, die meist mit einem anderen Material darunter thermisch verbunden sind. Somit kann erreicht werden, dass ein offener Rahmen oben wie unten verschlossen wird. Die Vorwärmung der bereits geschmolzenen Schicht kann vor oder nach dem Auftrag der neuen Metallpulverschicht erfolgen. Über eine Überwachung (Monitoring) des Volumenenergieeintrages und Erfassung der realen Schmelzzone und den daraus resultierenden Integrationsgrad kann wiederum der Volumenenergieeintrag zeitnah, lokal definiert, selbstregelnd angepasst werden. Für ein solches zeitnahes Monitoring und eine Regelung des Strahlschmelzprozesses ist dabei besonders vorteilhaft die Verwendung von zwei parallel anzuwendenden Messverfahren mit unterschiedlichen physikalischen Messprinzipien, die sich nicht gegenseitig beeinflussen, wie z. B. eine optische und eine akustische Detektion und Charakterisierung des laufenden Schmelzprozesses.

Als Funktionskomponente können vorzugsweise Aktoren und/oder Energiewandlerelemente, insbesondere elektrische, elektromagnetische, mechanoakustische, kinetische, thermische und andere Wandlersysteme eingesetzt werden.

Für die Integration einer Funktionskomponente mit umgebendem Formkörper mit unterschiedlichen Funktionalitäten ist eine variierende Abfolge von aufeinanderfolgenden Prozessschritten günstig. So ist es z. B. vorteilhaft, im Falle einer Integration einer Funktionskomponente in einen Formkörper, beispielsweise eines Implantates oder einer Prothese oder eines industriellen Bauteils oder einer multifunktional nutzbaren Sensorplattform zum Material-, Bauteil- und/oder Tragwerkmonitoring, den additiven Herstellungsprozess in mindestens zwei Schritte aufzuteilen, so dass der Formkörper aus mehreren Teilkörpern, die separat hergestellt werden, gefertigt werden kann. Der Fertigungsprozess des Formkörpers kann unterbrechungsfrei additiv mittels Strahlschmelzen erfolgen, bis der Aufnahmeraum für Funktionskomponente(n) mit Trägerstruktur und der Verbindungsschnittstelle, z. B. Spalt, Nut, Schlitz u. ä. aufgebaut ist und die eigentliche form-, kraft- und stoffschlüssige Verbindung erfolgen kann. Ist die Funktionskomponente durch einen vorhergehenden, separaten Einbettungsprozess bereits soweit thermisch geschützt, kann nach der Verbindung von Funktionskomponente und eines Teilkörpers der Formkörper mittels Strahlschmelzen fortgesetzt werden und mindestens ein weiterer mittels Strahlschmelzen hergestellter Teilkörper mit dem Teilkörper, der mit der Funktionskomponente über die Trägerstruktur bereits verbunden ist, stoffschlüssig verbunden werden. Die stoffschlüssige Verbindung kann beim Strahlschmelzen erreicht werden. Es ist aber auch ein Löten, Schweißen oder Schmieden möglich. Erfolgt die Einbettung der Funktionskomponente in den Formkörper über die Herstellung von Teilkörpern, stellen Teilkörper separate Halbzeuge oder Funktionseinheiten (z. B. ein monolithisch vollummantelter Teilkörper) dar. Teilkörper können an weitere Teilkörper oder andere Formkörper z. B. als Aktoren, Sensoren oder Monitoringsysteme angeschweißt, angelötet, eingegossen, mechanisch gefügt oder geklebt werden.

Wenn die Funktionskomponente nicht ohne Überstand in den Innenraum integriert werden kann bzw. der Form nach hinterschnittbehaftet sein muss, ist eine Anpassung der Strahlschmelz-Anlagentechnik notwendig. Erfindungsgemäß sollen hierbei manipulative, robotergesteuerte Werkstoffpulverzuführungssysteme sowie 3-D-bewegliche und/oder drehbare Fertigungstischelemente zur Aufnahme der Bauplattform und/oder 3-D-bewegliche Laserstrahlführungs- und -formungssysteme verwendet werden. Der besondere Vorteil einer 3-D-Führungsmöglichkeit von Bauplattform und/oder Werkstoffpulverzufuhr und/oder des Lasers besteht darin, dass somit kompliziertere Formkörper, sogar mit Hinterschneidungen, gestaltbar sind und eine bessere Einbettung bzw. Umbauung der Funktionskomponente erfolgen kann. Weiterhin vorteilhaft kann mit einer solchen 3-D-Führungsmöglichkeit von Bauplattform und/oder Werkstoffzufuhr und/oder des Lasers auch mit teilweisem und/oder vollständigem Überstand über die Pulverbettoberfläche beim Strahlschmelzen hinaus, d. h. Überstand in vertikaler Richtung, eingebettet bzw. umbaut werden. Ebenso vorteilhaft kann hierbei auch die Integration von keramischen oder metallischen Hilfs- und Haltestrukturen sein, welche temporär eingesetzt werden und nach dem Verschmelzen eines topischen Bereichs wieder entnommen werden können.

So kann die Funktionskomponente beispielsweise in eine vorher ebenfalls additiv gefertigte Hülse aus dem gleichen Werkstoff und mit dem gleichen Verfahren integriert werden. Funktionskomponente und Hülse können punktuell, linear oder flächig miteinander verbunden werden. Dabei kann es auch vorteilhaft sein, wenn diese Hülse ohne die Funktionskomponente separat stoffverdichtend über thermische Verfahren, wie z. B. Heiß-Isostatisches Pressen (HIP), vorbehandelt wird. Die Funktionskomponente kann anschließend nicht absolut form- und/oder kraftschlüssig in die Hülse eingebracht (Spiel, Knautschzone, Verformungszone) werden und nur über ihre Verbindungsschnittstellen zum Formkörper form-, kraft- und/oder stoffschlüssig verbunden sein. Es kann eine mechanische, nicht thermische Nachverdichtung des Formkörpers mit integrierter Funktionskomponente erfolgen, dies ist z. B. durch Kaltmassivumformung möglich, um beispielsweise eine optimal kraftabsorbierende Schicht oder Materialzone einzustellen, die die Funktionskomponente vor übermäßiger mechanischer Deformation schützt. Auf diese Weise kann beispielsweise der Formkörper nach der Integration der Funktionskomponente und fertiger additiver Herstellung und ggf. darauffolgender weiterer Fügeprozesse zur Gewährleistung einer notwendigen Festigkeit, Steifigkeit und Stabilität so verdichtet werden, dass die Funktionskomponente nicht funktionsschädigend deformiert wird. In diesem Falle ist auch eine lokal definierte Verdichtung in bestimmten Bereichen des Formkörpers, z. B. im definierten Abstand zur Verbindungsschnittstelle vorteilhaft möglich. Über die individuellen mechanischen Eigenschaften der separaten Volumina können z. B. räumlich Werkstoffeigenschaften eingestellt werden, sodass beispielsweise lasttragende Bereiche kraftaufnehmend so um den Funktionskomponentenbereich herum geführt werden können, ohne die Gesamtstabilität des Formkörpers und die Funktion der Funktionskomponente zu beeinträchtigen.

Für die Integration einer Funktionskomponente in einen Formkörper in Gestalt eines Implantates oder einer Prothese oder eines industriellen Bauteils oder einer multifunktional nutzbaren Sensorplattform zum Material-, Bauteil- und/oder Tragwerkmonitoring als Gesamtformkörper ist es z. B. vorteilhaft, wenn der Formkörper aus mindestens zwei segmentierten Teilkörpern hergestellt wird, die additiv hergestellt und additiv oder konventionell gefügt werden können. Die einzelnen Volumina des gesamten Formkörpers können vor dem Fügeprozess beispielsweise separaten thermischen und/oder mechanischen Behandlungsverfahren unterzogen werden, um gezielte Werkstoffeigenschaften einstellen zu können.

Vorteilhaft kann es sein, wenn der Formkörper aus mindestens zwei Teilkörpern besteht, die generativ gefertigt und nachträglich nach der Integration der Funktionskomponente in einen der Teilkörper zusammengefügt werden. Dies kann beispielsweise mittels Schweißen oder Schmieden erfolgen. Weiterhin vorteilhaft kann es sein, wenn der Formkörper aus mindestens zwei Teilkörpern besteht und nach der Integration der Funktionskomponente in den ersten Teilkörper alle weiteren Teilkörper auf den ersten Teilkörper generativ mittels Strahlschmelzen aufgebaut werden, bis der Formkörper vollständig hergestellt ist. Baut man additiv separat einen mittleren Teilkörper eines Formkörpers mit einer beidseitig offenen/durchbrochenen Kavität zur Aufnahme der Funktionskomponente mit den oben genannten Verbindungsschnittstellen (Nut, Schlitze, Spalte etc.) auf der Formkörperseite für die entsprechenden gegenüberstehenden Verbindungsschnittstellen der Trägerstruktur der Funktionskomponente (Feder, Anker etc.) auf, kann nach deren Einsatz, mechanischer Fixierung und Platzierung auf der Ober- oder Unterseite des mittleren Teilkörpers der jeweilige additive Fertigungsprozess fortgesetzt werden, wobei zuerst die jeweiligen oberen oder unteren Verbindungsschnittstellen der aneinander oder beieinander liegenden Verbindungsschnittstellen verbunden werden können. Die Aufnahme mit Funktionskomponente kann teilweise oder vollständig form-, stoff- und/oder kraftschlüssig segmentartig überbaut werden. Damit ist auf beiden Seiten der Funktionskomponente eine form-, kraft- und/oder stoffschlüssige Verbindung bezüglich des additiv hergestellten Teilkörpers möglich.

Am Formkörper oder einem Teilkörper für die Herstellung eines Formkörpers kann eine zu dem/den Konturelement(en) komplementäre Kontur ausgebildet werden, so dass bei einem Einsetzen von Funktionskomponente mit Trägerstruktur dadurch zumindest eine formschlüssige Verbindung erhalten werden kann. Bei entsprechender Dimensionierung von Konturelement(en) und Kontur(en) im Formkörper oder einem Teilkörper kann zusätzlich auch eine kraftschlüssige Verbindung in Form einer Presspassung erreicht werden. Ganz wichtig ist dabei ist, dass das mindestens eine Konturelement der Trägerstruktur der Funktionskomponente wenigstens eine stoffschlüssige Verbindung zum Formkörper aufweist, um die aktorische und/oder sensorische Funktionalität des Verbundkörpers sicher zu gewährleisten.

Ein wesentlicher Vorteil der Erfindung besteht in dem Verzicht auf beispielsweise Deckelkonstruktionen, Schraubenstrukturen oder andere konstruktive Ein- und Vorspannungsschritte im additiven Herstellungsverfahren, wenn die Vor- und Einspannung z. B. eines Piezoaktors als Funktionskomponente nicht direkt über die Gestaltung der Aufnahme im Formkörper, sondern über die Gestaltung der z. B. ebenfalls besonders vorteilhaft im Strahlschmelzen vorgefertigten Trägerstruktur erfolgt.

Es kann vorteilhaft sein, wenn die Funktionskomponente in einen Teilkörper des Formkörpers als Halbzeug eingebettet wird, um anschließend als Halbzeug/Teilkörper in die Herstellung des Verbundkörpers integriert zu werden.

Wird als umgebendes Halbzeug für eine Funktionskomponente z. B. eine Hülse mit an ihrer Oberfläche befindlichen Erhebungen und Vertiefungen eingesetzt, kann die Verbindung zum Formkörper nur im Bereich von Erhebungen erfolgen. Es können so großflächig ungefüllte Hohlräume mit einem wärmeisolierenden Charakter erhalten werden, sodass Wärmeenergie aus der Funktionskomponente nur erschwert in den umgebenden Formkörper gelangen und umgekehrt vom Formkörper zur Funktionskomponente gelangen kann (Thermoskanneneffekt). Der gleiche Effekt kann auch realisiert werden, indem die Funktionskomponente gewölbeartig überbaut und so ein Hohlraum ausgebildet wird und die form-, kraft- und/oder stoffschlüssige Integration nur im Bereich der Verbindungsschnittstellen, z. B. einer Feder-Nut-Verbindung (Interfaces), erfolgt.

Um eine funktionserhaltende Einbettung einer Funktionskomponente (Aktor oder Sensor) zu ermöglichen, ist der Energieeintrag beim Strahlschmelzprozess lokal zu begrenzen. Ferner ist darauf zu achten, dass der Temperaturgradient zwischen aktuell zu schmelzenden sowie bereits geschmolzenen und verfestigten Bereichen nicht zu hoch ist, um somit Spannungen zu reduzieren. Ebenso kann ein örtlich-zeitlich angepasster/variierender Energieeintrag vorteilhaft sein. Dies kann beispielsweise in der Ausführung der direkten, d. h. darauf aufbauenden, oder der gewölbeartigen Überbauung der Funktionskomponente sinnvoll sein, indem beispielsweise der Energieeintrag des Energiestrahls mit wachsendem Abstand zur Funktionskomponente erhöht und in der Nähe der Funktionskomponente reduziert wird (geringer Abstand = niedriger Energieeintrag, großer Abstand = hoher Energieeintrag). Die Dimensionierung des Energieeintrags sollte dabei so gewählt werden, dass der Stoffschluss noch gegeben ist. Erfordert der Stoffschluss trotzdem einen höheren Energieeintrag, so kann der darunter liegende temperatursensible Bereich, in dem eine Funktionskomponente angeordnet ist, mit einer stärkeren wärmeverteilenden Schicht ummantelt werden, um den thermischen Eintrag auf einen größeren Bereich im Form- oder Teilkörper mit der Funktionskomponente zu verteilen und damit lokal definiert zu senken. Je nach Art und Wirkungsweise der Funktionskomponente und der Gestaltung des umgebenden Formkörpers sollten das thermische Schutzmantelsystem im Teilkörper mit Funktionskomponente sowie der Energieeintrag bei der additiven Fertigung miteinander abgestimmt werden. Dies kann auch konstruktiv berücksichtigt werden, indem der Energieeintrag beim stoffschlüssigen Verschweißen von Nut und Feder entsprechend beeinflusst wird. Aufgrund des hohen Werkstoffanteils im äußeren eine Rahmenfunktion erfüllenden Bereich des Formkörpers oder eines Teilkörpers mit Funktionskomponente kann der überwiegende Anteil des Wärmeenergieeintrags auf diesen Rahmenbereich und nicht auf die Funktionskomponente übertragen werden. Wärme kann damit auch abgeleitet werden, d. h. von der Trägerstruktur der Funktionskomponente ferngehalten werden. Vorteilhaft kann die Prozessführung mit vorgewärmtem Pulver und Bauteil erfolgen. Dies kann beispielsweise über eine Bauplattformheizung, Strahlungsheizung, Induktionsheizung oder Baukammerheizung geschehen.

Weitere vorteilhafte Ausführungen sind den Unteransprüchen zu entnehmen.

Unter "spezifischer Maximaltemperatur" soll in dieser Anmeldung verstanden sein, dass das jeweilig betroffene Element mit höchstens einer Temperatur belastet wird, so dass dieses Element in seinen Funktionen keinen Schaden erleidet, d. h. nach Beendigung der Belastung mit einer Temperaturerhöhung entsprechend seines Einsatzzweckes voll funktionstüchtig ist.

Mit dieser Erfindung wird es ermöglicht, Spannungsänderung innerhalb des Formkörpers zu detektieren. Dafür werden vorteilhafterweise mindestens eine aktorisch wirkende Funktionskomponente und eine sensorisch wirkende Funktionskomponente in den Formkörper integriert, wobei die mindestens eine aktorisch wirkende Funktionskomponente den Formkörper zum Schwingen anregt (Körperschall) und die mindestens eine sensorisch wirkende Funktionskomponente die Schwingungen detektiert. Auf diese Weise können zeitlich veränderliche Spannungszustände innerhalb des Formkörpers detektiert werden. So wird es möglich, den mechanischen Bauteilzustand zu monitoren und das Bauteilversagen frühzeitig vorherzusagen.

Nachfolgend soll die Erfindung an Ausführungsbeispielen unter Zuhilfenahme von Zeichnungen näher erläutert werden. Hierbei zeigen:
- Figur 1: ein Beispiel eines erfindungsgemäß hergestellten Elements, bei dem eine Funktionskomponente mittels einer Trägerstruktur form-, kraft- und stoffschlüssig mit einem Formkörper verbunden ist, in zwei Ansichten;
- Figur 2: ein Beispiel einer mit einem Formkörper form-, kraft- und stoffschlüssig verbundenen Trägerstruktur, bei dem auf die Darstellung der Funktionskomponente verzichtet worden ist;
- Figur 3a: ein Beispiel einer Funktionskomponente mit umgebendem Schichtsystem in einer Schnittdarstellung;
- Figur 3b: einen Verbund von Funktionskomponente mit Trägerstruktur in Seitenansicht;
- Figur 3c: mögliche Strahlschmelzzonen;
- Figur 3d: eine Funktionskomponente mit Trägerstruktur, eingesetzt in einen Formkörper als Ausschnitt;
- Figur 4a: zwei Ansichten einer Trägerstruktur mit Konturelementen;
- Figur 4b-d: jeweils zwei Ansichten von Erhebungen (punkt- oder linienförmig, axial oder radial angeordnet) für die form- und/oder stoffschlüssige Verbindung;
- Figur 5: ein Beispiel, bei dem eine Funktionskomponente von einem Gewölbebogen eines Formkörpers umbaut worden ist, in mehreren Ansichten;
- Figur 6: ein Beispiel, bei dem eine Funktionskomponente von zwei Gewölbebögen eines Formkörpers umbaut worden ist, in mehreren Ansichten;
- Figur 7a: eine Endoprothese als dreiteilige Ausführungsform;
- Figur 7b: eine Endoprothese als zweiteilige Ausführungsform;
- Figur 8: eine schematische Darstellung der Vorschubbewegung eines zum Strahlschmelzen genutzten Energiestrahls in der Strahlschmelzzone (Verbindungsbereich des Konturelementes der Funktionskomponente und des Formkörpers);
- Figur 9: schematische Darstellungen für Bestrahlungsmuster bei der Herstellung eines Formkörpers;
- Figur 10: mögliche Bestrahlungsmuster eines bewegten Energiestrahles mit örtlichzeitlich variablem Energieeintrag bei der Ausbildung von Deckschichten über einer Funktionskomponente und
- Figur 11: weitere Möglichkeiten für eine örtlich-zeitliche variable Bestrahlung mit einem Energiestrahl bei der Ausbildung von Deckschichten oberhalb eines bereits in den Formkörper eingesetzten Konturelementes der Funktionskomponente.

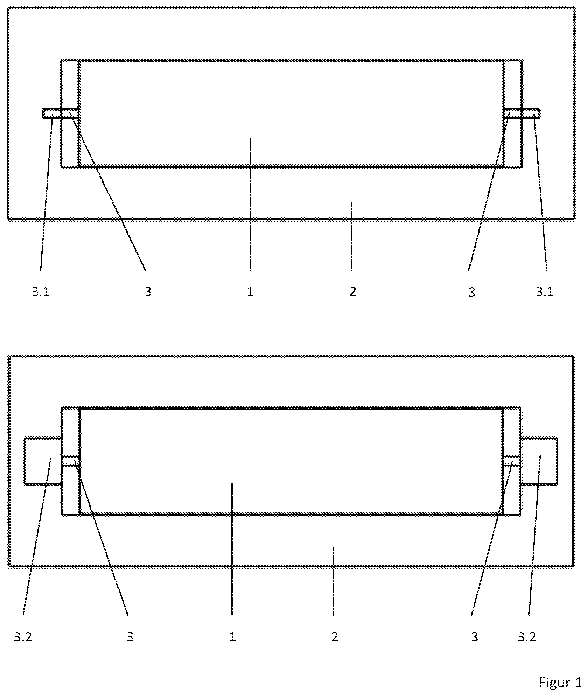

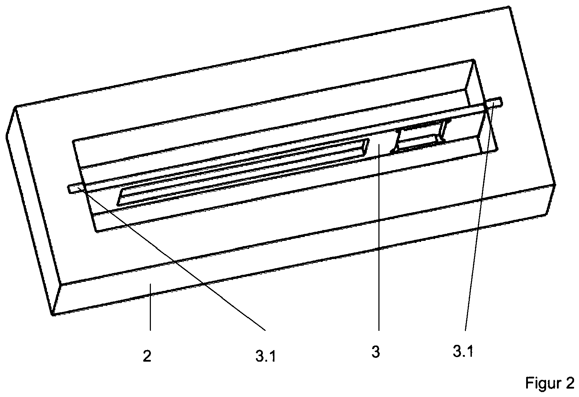

In Figur 1 sind Schnittdarstellungen in zwei Ansichten eines Verbundkörpers mit einer Funktionskomponente 1, die mit einem Formkörper 2 im Bereich von Konturelementen 3.1 einer Trägerstruktur 3 form- und stoffschlüssig verbunden ist, gezeigt. Die Trägerstruktur 3 ist dabei vorzugsweise aus dem gleichen Metall gebildet wie der Formkörper 2, der aus Metallpulver schichtweise generativ durch Strahlschmelzen hergestellt worden ist. Die Funktionskomponente 1 ist dabei bevorzugt formschlüssig mit der Trägerstruktur 3, die mit Durchbrechungen/Aussparungen versehen ist (s. Figur 2), verbunden. Die Strahlschmelzzone 3.2 markiert die Integrationszonen, wo über Strahlschmelzen die stoffschlüssige Verbindung zwischen den Konturelementen 3.1 der Trägerstruktur 3 der Funktionskomponente 1 und dem Formkörper 2 hergestellt wird.

Gemäß Figur 2 sind an den zwei gegenüberliegenden Stirnseiten der Trägerstruktur 3 Konturelemente 3.1 vorhanden, die bei der generativen Herstellung mit dem Werkstoff des Formkörpers 2 umschlossen werden, so dass ein form- und stoffschlüssiger Verbund im Bereich der Konturelemente 3.1 erreicht werden kann. Bei der Herstellung des Formkörpers 2 und der Ausbildung dieser Verbindung in diesem Bereich ist es erforderlich, den Energieeintrag so zu beeinflussen, dass eine Beeinträchtigung der Funktionskomponente 1 durch Wärme, also zu hohe Temperaturen, vermieden werden kann. Der Energieeintrag muss so limitiert und beeinflusst werden, dass eine für eine vorgebbare Funktionskomponente 1 spezifische Maximaltemperatur an der jeweiligen Funktionskomponente 1 vermieden wird. Dabei sollte auch die thermische Leitfähigkeit der Trägerstruktur 3 berücksichtigt werden.

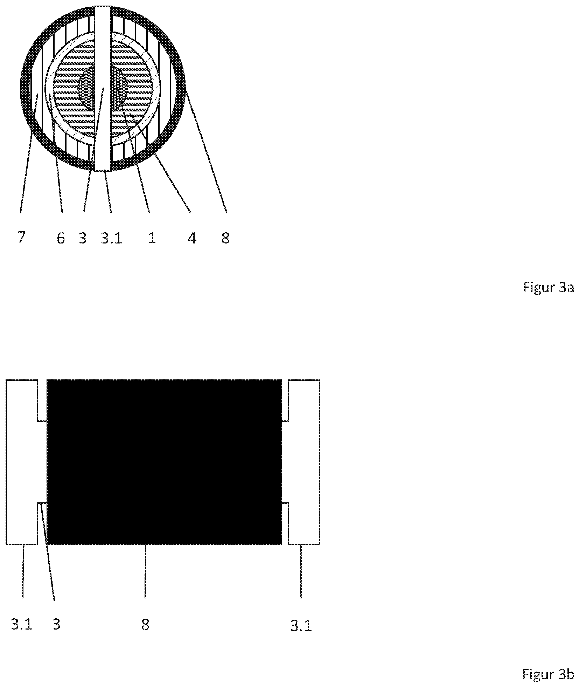

In Figur 3a ist eine zur Integration in einen Formkörper 2 vorgesehene Funktionskomponente 1 in einer Schnittdarstellung mit ihren mehreren Schichten gezeigt. Die Funktionskomponente 1 ist mit einer Trägerstruktur 3 (nicht dargestellt), die an beiden Seiten Konturelemente 3.1 aufweist, verbunden. Die Funktionskomponente 1 ist von einer ersten keramischen Schicht 4 umschlossen, die beispielsweise aus Zirkon- oder Aluminiumoxid gebildet ist. Auf dieser ersten keramischen Schicht 4 ist eine weitere keramische Schicht 6 ausgebildet, die vorteilhafterweise auch aus Keramiken, wie z. B. Knochenzement, gebildet sein kann. Die Schichten 4 und 6 bilden thermische Isolatoren und schützen die Funktionskomponente 1 vor zu großem Wärmeeintrag beim späteren Strahlschmelzen. Auf der weiteren keramischen Schicht 6 ist eine metallische Schicht 7 vorhanden, die aus Molybdän bestehen kann. Die metallische Schicht 7 wiederum wird von einer an den zwei Stirnseiten, an denen die Konturelemente 3.1 herausragen, offenen Hülse 8 aus Titan umschlossen. Titan ist bei diesem Beispiel gewählt, weil der Formkörper 2, in den der Verbund Funktionskomponente 1 mit Trägerstruktur 3 integriert werden soll, ebenfalls aus Titan besteht.

Sämtliche Schichten, die einerseits zur Wärmeisolation und andererseits zur besseren Wärmeverteilung oder Wärmespeicherung vorhanden sind, können mittels thermischen Spritzens, insbesondere Plasmaspritzens ausgebildet werden. So kann beispielsweise eine temperaturverteilende metallische Schicht 7 aus Molybdän mit einer Schichtdicke im Bereich von 50 µm bis 200 µm aufgetragen werden. Als Schutz- und Kühlgas kann vorzugsweise Argon oder ein anderes geeignetes inertes Gas eingesetzt werden. In diesem Falle wäre ein Vakuum für das thermische Spritzen nicht unbedingt notwendig.

Das Kühlgas könnte auch Stickstoff sein. Das thermische Spritzen als ein thermisches Beschichtungsverfahren könnte aber auch unter Vakuumbedingungen durchgeführt werden.

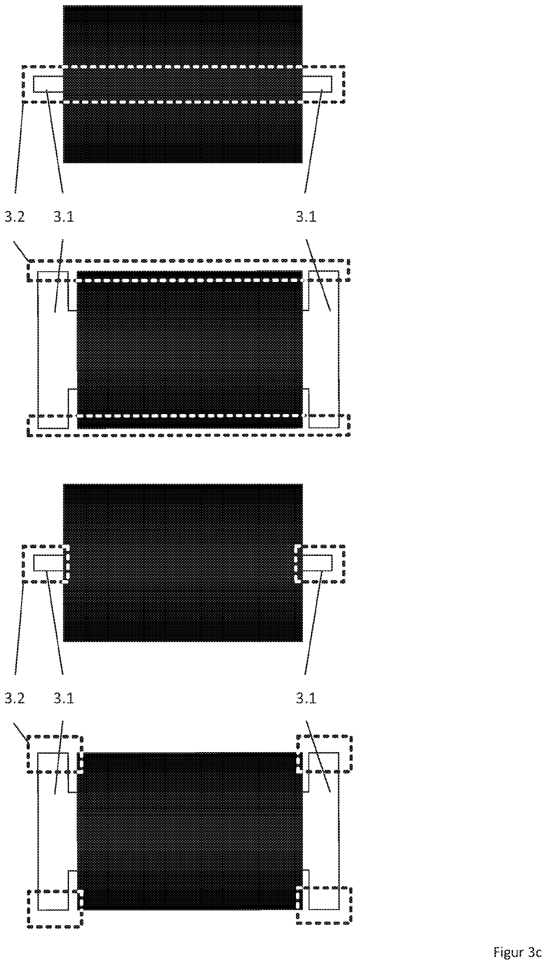

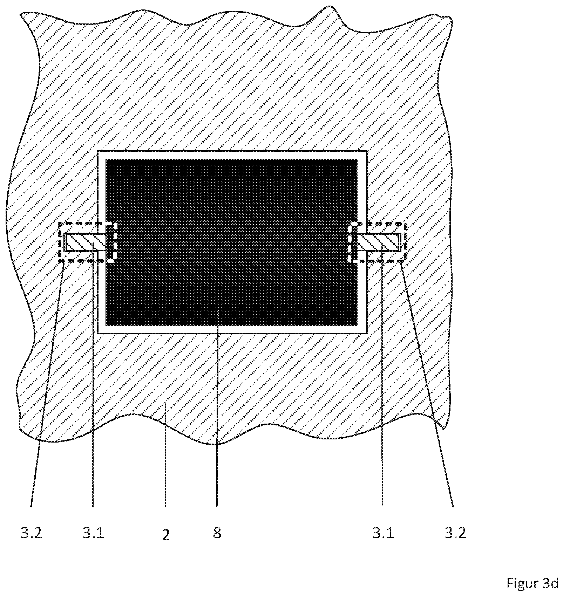

In Figur 3b ist die in Figur 3a gezeigte Funktionskomponente 1 mit seinen Konturelementen 3.1 der Trägerstruktur 3 in einer Seitenansicht gezeigt. In Figur 3c sind die verschiedenen möglichen Strahlschmelzzonen 3.2 zum stoffschlüssigen Verbinden mit den Konturelementen 3.1 aufgezeigt (dargestellt sind zwei Varianten in Draufsicht und Seitenansicht). Figur 3d zeigt die in den Formkörper 2 eingesetzte Funktionskomponente 1, wobei nur die Hülse 8 und die Konturelemente 3.1 in den Strahlschmelzzonen 3.2 dargestellt sind.

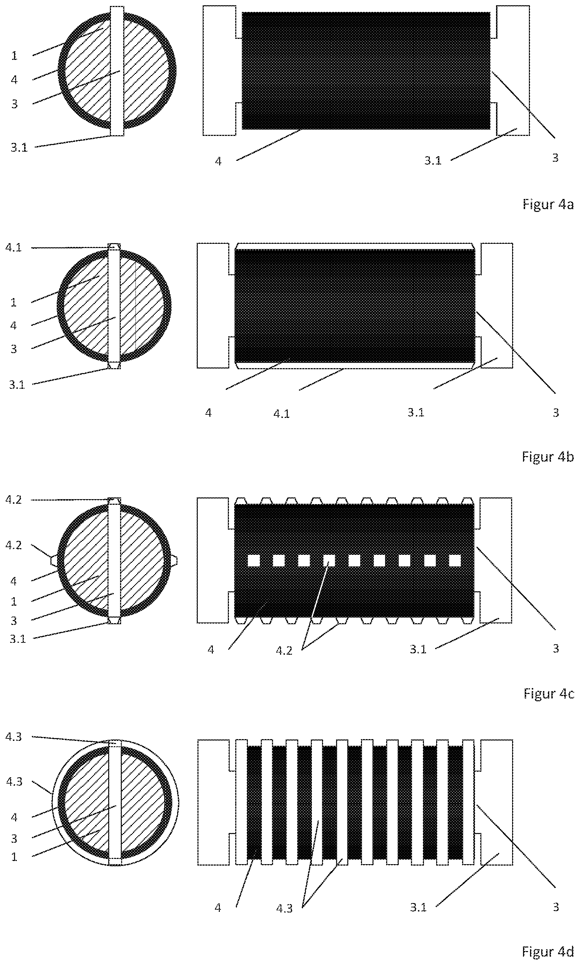

Die Figuren 4a-d zeigen mehrere Beispiele in jeweils zwei Ansichten, bei denen eine Funktionskomponente 1 im Bereich zwischen den nach außen ragenden Konturelementen 3.1 der Trägerstruktur 3 von einer äußeren keramischen Schicht 4 umgeben ist.

In Figur 4a ist die keramische Schicht 4 ein einfacher Hohlzylinder. Das in Figur 4b gezeigte Beispiel unterscheidet sich vom Beispiel nach Figur 4a dadurch, dass auf die keramische Schicht 4 zwei Längsnoppen 4.1 über die gesamte Länge aufgebracht wurden. Gemäß Figur 4b liegen diese Längsnoppen 4.1 genau zwischen den Konturelementen 3.1, was jedoch nicht zwingend sein muss. In weiteren Ausführungen können auch mehr als zwei Längsnoppen 4.1 über den Umfang der keramischen Schicht 4 verteilt angeordnet sein. Bei dem in Figur 4c gezeigten Beispiel sind Einzelnoppen 4.2 in diskreten Abständen auf der gesamten Oberfläche der keramischen Schicht 4 angeordnet. In Figur 4d sind mehrere Radialnoppen 4.3 kreisringförmig über die gesamte Oberfläche der keramischen Schicht 4 verteilt angeordnet. Sämtliche Noppen 4.1, 4.2, 4.3 dienen der besseren stofflichen Integration zum Formkörper 2 hin und erfüllen gleichzeitig die Funktion einer Abstandshaltung, wenn im weiteren Prozedere zwischen keramischer Schicht 4 und Formkörper 2 ein thermisch isolierender Raum bewusst mit eingebaut wird. Im Querschnitt sind die Noppen 4.1, 4.2, 4.3 vorzugsweise trapezförmig, da so eine optimale stoffschlüssige Verbindung zum Formkörper 2 hergestellt werden kann. Auch kann eine gewölbeförmige Überbauung von Noppe zu Noppe im Bereich von Strahlschmelzzonen 3.2 somit optimal realisiert werden. Je nach Anwendungsfall können die Noppen 4.1, 4.2, 4.3 vorteilhafterweise aus Keramik oder Metall bestehen. Damit können Abstandshaltungs- und Anschmelzungsfunktionen realisiert werden.

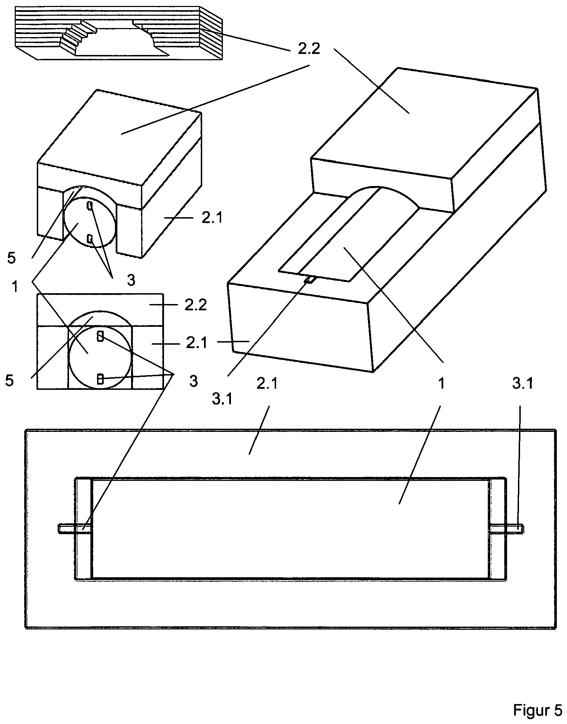

Die Figur 5 zeigt in mehreren Darstellungen ein Beispiel, bei dem eine Funktionskomponente 1 in einen bereits vorgefertigten Teilkörper 2.1 eingesetzt worden ist. Darüber wird ein weiterer Teilkörper 2.2 generativ durch schichtweises Strahlschmelzen aus dem gleichen Werkstoff wie der erste Teilkörper 2.1 gefertigt und umschließt somit die Funktionskomponente 1. Bei diesem Beispiel ist die Funktionskomponente 1 mit der nicht dargestellten Trägerstruktur 3 und all ihren ebenfalls nicht näher dargestellten Schichten bündig in den Teilkörper 2.1 eingelegt, so dass der Teilkörper 2.2 gewölbeförmig die Funktionskomponente 1 überbaut. Gemäß Figur 5 verbleibt zwischen der äußeren Oberfläche der Funktionskomponente 1 und dem Teilkörper 2.2 ein Hohlraum 5. Mit diesem Hohlraum 5 kann eine thermische Isolation erreicht werden, so dass Wärme bei der generativen Herstellung des Teilkörpers 2.2 nicht oder mit verminderter Temperatur die Funktionskomponente 1 erreicht. Der Hohlraum 5 kann auch mit nicht aufgeschmolzenem Pulver gefüllt sein. Grundsätzlich kann der Formkörper 2 generativ in einem Zug aufgebaut werden, d. h. erst der erste Teilkörper 2.1 mit gleichzeitiger Einbindung der Funktionskomponente 1 mit seinen Konturelementen 3.1 oder Noppen 4.1, 4.2, 4.3 und gleich anschließend der weitere Aufbau der Teilkörper 2.2, so dass in einem Strahlschmelzprozess der Formkörper 2 generiert wird. Die form- und stoffschlüssige Verbindung von Trägerstruktur 3 und Formkörper 2 kann wie bei den Beispielen nach den Figuren 1 und 2 ausgebildet werden.

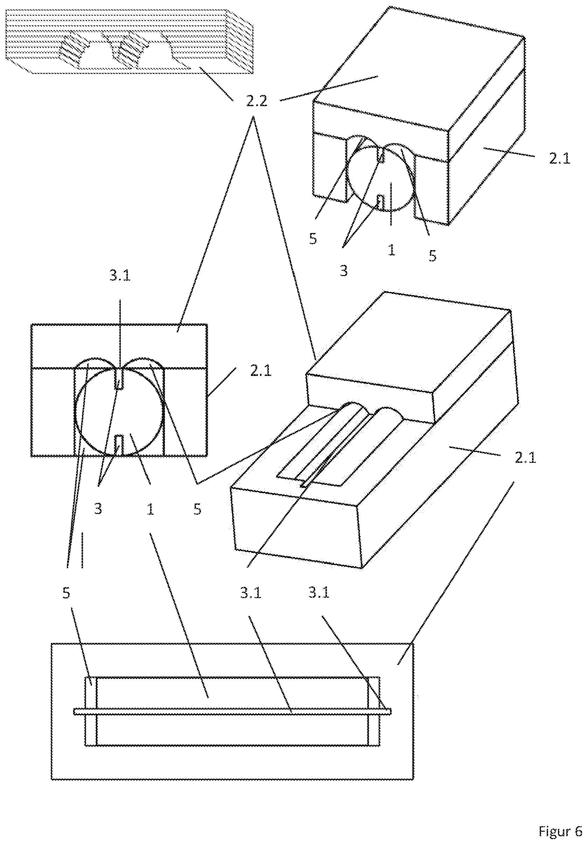

Das in Figur 6 gezeigte Beispiel unterscheidet sich vom Beispiel nach Figur 5 dadurch, dass am Teilkörper 2.2 zwei Gewölbebögen ausgebildet werden, wobei der Steg zwischen den beiden Gewölbebögen für eine zumindest stoffschlüssige Verbindung mit der Trägerstruktur 3 genutzt werden kann. Siehe dazu auch Figur 3c, die ersten beiden Abbildungen, in denen die Strahlschmelzzonen 3.2 eingezeichnet sind. Eine nicht dargestellte Variante gemäß Figur 6 ergibt sich analog dadurch, dass der Steg der Gewölbebögen über eine stoffschlüssige Verbindung zu einer angeordneten Längsnoppe 4.1 aufgebaut wird.

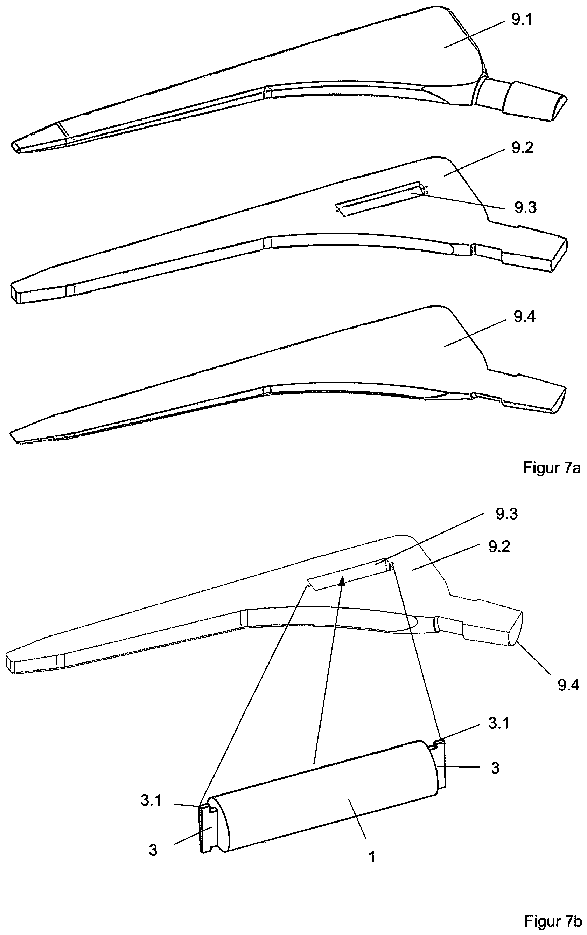

Figuren 7a und b zeigen eine Endoprothese, als ein Beispiel eines Formkörpers 2, in die eine Funktionskomponente 1 mit Trägerstruktur 3 integriert werden kann. Dabei kann die Endoprothese ebenfalls durch Strahlschmelzen generativ hergestellt werden. Die Endoprothese kann aus Titan oder einer Titanlegierung oder aus einer Kobalt-Chrom-Legierung hergestellt werden.

Wie aus Figur 7a hervorgeht, kann die Endoprothese in mehreren Stufen generativ hergestellt werden. Wie durch die mittlere Abbildung gezeigt, verbleibt zunächst im Mittelteil 9.2 eine Aussparung 9.3, in die die Funktionskomponente 1 mit ihrer Trägerstruktur 3 und den Konturelementen 3.1 gemäß Figur 7b eingesetzt und formschlüssig verbunden werden kann. Anschließend wird auf das Mittelteil 9.2 das Oberteil 9.1 mittels Strahlschmelzen aufgebaut, der hergestellte Verbund aus Mittelteil 9.2 und Oberteil 9.1 um 180° gedreht und anschließend das Unterteil 9.4 mittels Strahlschmelzen stoffschlüssig auf den Verbund aus Mittelteil 9.2. und Oberteil 9.1 aufgebaut. Zwischen den einzelnen Aufbauschritten können die jeweiligen Teil- und Verbundkörper auch konventionellen Bearbeitungsschritten, wie z. B. Schleifen, unterzogen werden. Die so hergestellte Endoprothese besteht somit aus einem die Funktionskomponente 1 vollständig umschließenden monolithischen Formkörper 2, wobei Oberteil 9.1, Mittelteil 9.2 und Unterteil 9.4 durch den gewählten Herstellungsprozess an ihren Grenzflächen gleichsam stoffschlüssig verschmolzen sind. Es kann sich eine mechanische, nicht thermische Nachverdichtung der Endoprothese mit integrierter Funktionskomponente 1 anschließen. Dies ist z. B. durch Kaltmassivumformung um eine optimal kraftabsorbierende Schicht oder Materialzone, die die Funktionskomponente 1 vor übermäßiger mechanischer Deformation schützt, möglich. Diese Nachbehandlung kann ganzheitlich oder auch nur selektiv in Teilbereichen der Endoprothese erfolgen. Auf diese Weise kann die Endoprothese nach der Integration der Funktionskomponente 1 und fertiger additiver Herstellung und ggf. darauffolgenden weiteren Fügeprozessen zur Gewährleistung einer notwendigen Festigkeit, Steifigkeit und Stabilität so verdichtet werden, dass die Funktionskomponente 1 nicht funktionsschädigend deformiert wird. In diesem Falle ist auch eine lokal definierte Verdichtung in bestimmten Bereichen der Endoprothese, z. B. im definierten Abstand zur Verbindungsschnittstelle vorteilhaft möglich. Über die individuellen mechanischen Eigenschaften der separaten Volumina können räumlich Werkstoffeigenschaften eingestellt werden, sodass lasttragende Bereiche kraftaufnehmend so um den Funktionskomponentenbereich herum geführt werden können, ohne die Gesamtstabilität des Formkörpers 2 und die Funktion der Funktionskomponente 1 zu beeinträchtigen. In Figur 7b ist eine Variante dargestellt, wobei der Formkörper 2 aus einem Unterteil 9.4 und einem Oberteil 9.1 (in Figur 7a dargestellt) besteht. Im Unterschied zu Figur 7a wird kein separates Mittelteil 9.2 mit einer Aussparung 9.3 hergestellt. Das Unterteil 9.4 wird hierbei mit einer Aussparung 9.3 bis auf die Höhe des wegfallenden Mittelteils 9.2 (siehe Figur 7a) aufgebaut. Die Aussparung 9.3 ist nur nach oben hin offen gestaltet, sodass die Funktionskomponente 1 formschlüssig in die Aussparung 9.3 eingebracht werden kann, wobei eine kraft- und stoffschlüssige Integration der Trägerstruktur 3 der Funktionskomponente 1 mit dem Unterteil 9.4 erfolgt. Abschließend folgt der Aufbau des Oberteils 9.1 auf das Unterteil 9.4 mit stoffschlüssig integrierter Funktionskomponente 1.

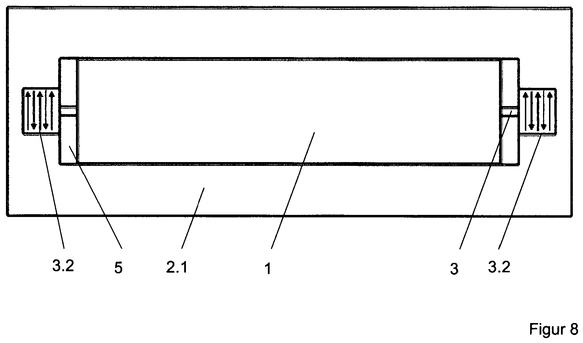

Mit Figur 8 soll verdeutlicht werden, wie zweckmäßigerweise die Vorschubbewegung eines Brennflecks eines Energiestrahls im Bereich der Strahlschmelzzone 3.2 erfolgen kann, um das Konturelement 3.1 stoffschlüssig mit dem Teilkörper 2.1 zu verbinden. Es besteht die Möglichkeit, die Bestrahlung in allen Schichten der Strahlschmelzzone 3.2 so in Spuren mit jeweils alternierend wechselnder entgegengesetzter Vorschubbewegungsrichtung des Brennflecks durchzuführen. Als Alternative können vorzugsweise auch eine bestimmte vorgebbare Anzahl, beispielsweise zehn Schichten, die unmittelbar über den Konturelementen 3.1 ausgebildet werden, so bestrahlt werden und anschließend daran kann dann beliebig bestrahlt werden. Es soll jedoch generell vermieden werden, dass über thermische Leitung über die Trägerstruktur 3 die spezifische Maximaltemperatur an der Funktionskomponente 1 überschritten wird.

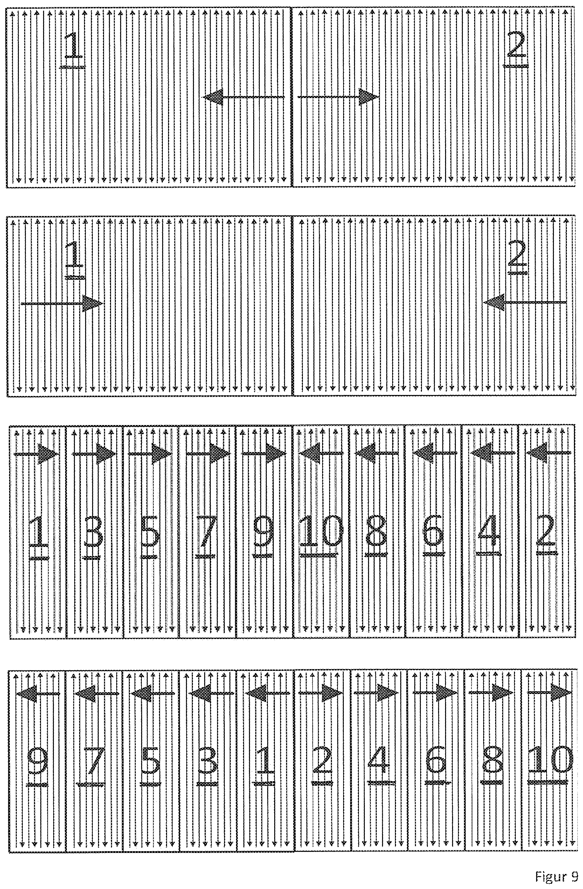

Mit Figur 9 soll verdeutlicht werden, wie die Vorschubbewegung eines Brennflecks eines Energiestrahls als Bestrahlungsmuster gewählt werden kann, wenn Schichten oberhalb einer Funktionskomponente 1 bestrahlt werden und ein geschlossener Formkörper 2 um die jeweilige Funktionskomponente 1 durch Strahlschmelzen hergestellt wird. Mit den Ziffern 1 bis 10 ist die jeweilige Reihenfolge der Bestrahlung von Segmenten der jeweiligen Schichten und mit den Pfeilen die Richtung der Vorschubbewegung des Brennflecks angegeben.

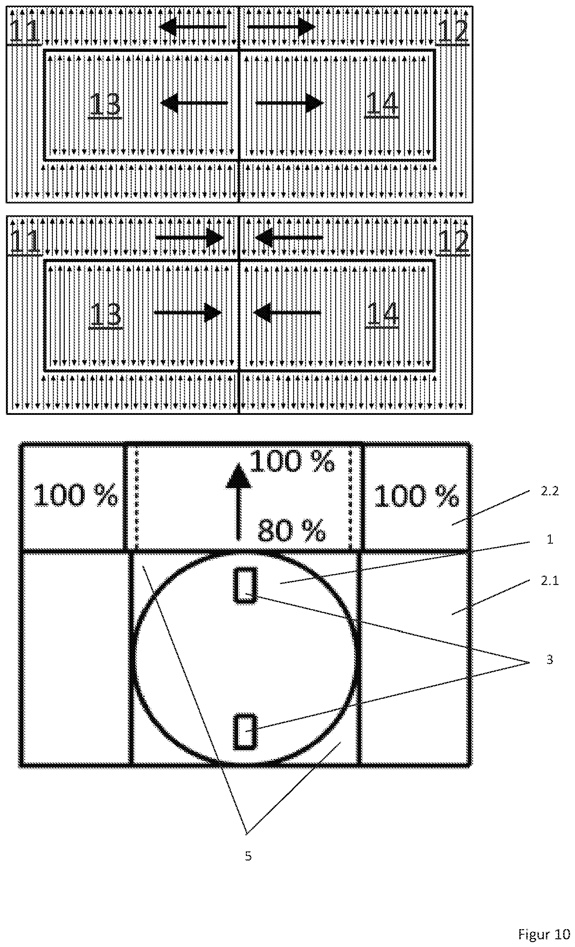

Figur 10 soll verdeutlichen, wie ein auf 80 % reduzierter Energieeintrag beim Strahlschmelzen in Bereichen 13 und 14 erreicht werden kann, die nah an einer Funktionskomponente 1 oder thermisch empfindlichen Teilen oder Bereichen einer Funktionskomponente 1 liegen und/oder die durch thermische Leitung besonders gefährdet sind. Dabei sollte ein Abstand von der Oberfläche der Funktionskomponente 1 eingehalten werden, bei dem die spezifische Maximaltemperatur durch thermische Leitung an der Funktionskomponente 1 vermieden wird. Es wird deutlich, dass der Teilkörper 2.2 in Bereichen 11 und 12, die zwar oberhalb, aber neben oder sogar in einem Abstand zur Funktionskomponente 1 angeordnet sind, mit einem 100 %-igen Energieeintrag hergestellt werden kann. Die unmittelbar oberhalb der Funktionskomponente 1 angeordneten Bereiche 13 und 14 des Teilkörpers 2.2 werden dagegen mit reduziertem Energieeintrag von hier lediglich maximal 80 %, der mit steigendem Abstand wieder auf 100 % geregelt werden kann, hergestellt. Die Reduzierung des Energieeintrags auf unterschiedlichen Niveaus kann zudem auch vorteilhaft innerhalb einer Schicht erfolgen.

In den oberen Abbildungen von Figur 10 sind alternativ bevorzugte Vorschubbewegungsrichtungen eines bewegten Brennflecks eines Energiestrahls in bestimmten Bereichen mit Pfeilen in einer Draufsicht dargestellt. In den Bereichen 11 und 12 kann ein vollständiger 100 %-iger Energieeintrag erfolgen, wohingegen zumindest im Nahbereich zu der Funktionskomponente 1 in den Bereichen 13 und 14 der Energieeintrag auf 80 % reduziert werden sollte.

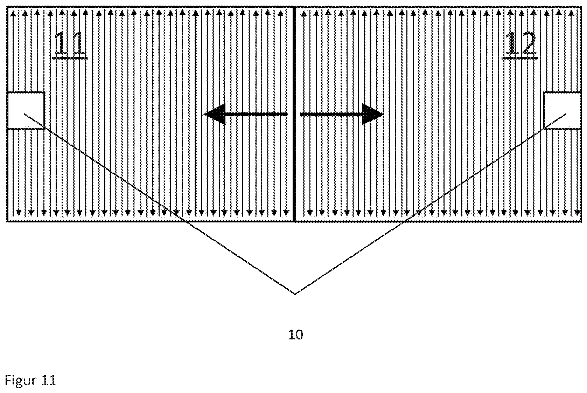

Mit Figur 11 soll verdeutlicht werden, dass bei der Herstellung von Teilkörpern 2.1 in Bereichen 10, die ober- und/oder unterhalb einer ausgebildeten oder auszubildenden form- und stoffschlüssigen Verbindung zwischen Konturelementen 3.1 und Formkörper 2 angeordnet sind, der Wärmeeintrag durch eine andere Vorschubbewegung eines Brennflecks eines Energiestrahls als in den Bereichen 11 oder 12 durchgeführt werden kann.

Bei der Herstellung eines Verbundkörpers mittels Strahlschmelzen kann mit Laserleistungen im Bereich zwischen 100 W - 2000 W, Vorschubgeschwindigkeiten des Brennflecks eines Laserstrahls im Bereich 500 mm/s - 5000 mm/s gearbeitet werden. Es sollten Schichtdicken einzelner Pulverschichten, die sukzessive aufgebracht und mit denen jeweils ein lokal definierter generativer Aufbau erfolgt, im Bereich 20 µm - 200 µm eingehalten werden. Der Abstand zwischen nebeneinander ausgebildeten Schmelzspuren jeweils von Mitte zu Mitte dieser Schmelzspuren wird als Hatchabstand bezeichnet und sollte 50 µm bis 500 µm betragen. Die Volumenenergiedichten sollten im Bereich 10 J/mm³ - 200 J/mm³ gehalten werden, damit entsprechende Leistungsdichten im Brennfleck beim Aufschmelzen des jeweiligen Pulvers erreicht werden können.

So kann der Werkstoff TiAl6V4 beispielsweise mit einer Laserleistung von 100 W, einer Vorschubgeschwindigkeit des Brennflecks eines Laserstrahls von 600 mm/s, bei Einhaltung einer Pulverschichtdicke von jeweils 30 µm, einem Hatchabstand von 105 µm und einer Volumenenergiedichte von 53 J/mm³ gearbeitet werden, wenn ein 100 %-iger Energieeintrag gemäß dem in Figur 10 gezeigten Beispiel erreicht werden soll.

Ist bei einer Aktor-Sensor-Wandler-Variante, z. B. bei einem Thermoaktor als Funktionskomponente 1 zur thermischen oder thermoelektrischen oder elektrochemischen Aktivierung von weiteren, agglomerierten Formgedächtnisaktoren eine freischwingende Funktionskomponente 1 nicht notwendig oder nicht vorteilhaft, so kann bzw. sollte die Funktionskomponente 1 auch an ihrer Oberfläche form- und stoffschlüssig mit dem umgebenden Formkörper 2 verbunden bzw. angebunden werden. In einer derartigen Ausführungsform wird die keramisch ummantelte Funktionskomponente 1 mit einer zum Formkörper 2 stoffgleichen Titanschicht ummantelt, die dann mittels Strahlschmelzen mit der stoffgleichen Titanlegierung des Formkörpers 2 aufgeschmolzen und damit verbunden werden kann. Um dabei ungewünschte Titanoxid- und/oder Nitritbildungen zu vermeiden, die den Form- und Stoffschluss wegen mangelnder Kompatibilität verhindern bzw. flächendeckend gefährden, sollte der thermische Spritzvorgang unter Vakuum erfolgen.

Ersatzweise kann die keramisch ummantelte Funktionskomponente 1 mit einer definiert rauen Titanschicht von bis zu 150 µm Dicke thermisch umspritzt werden und dann in eine bereits vorher additiv gefertigte Hülse 8 aus einem stoffgleichen Material bzw. Legierung, insbesondere Titan oder eine Titanlegierung geschoben werden, die eventuell vorher thermisch nachbehandelt und verdichtet wurde. Damit wäre dann auch ohne Vakuumspritzen ein optimaler Stoff-, Form- und/oder Kraftschluss beim nachfolgenden integrierenden Strahlschmelzen möglich. Die Hülse 8 kann auch aus Fertigungs- und Montagegründen vorzugsweise längs geschlitzt ausgeführt sein.

Um die thermisch empfindliche, keramisch ummantelte Funktionskomponente 1 (Aktor, Sensor und/oder ein anderes Wandlerelement) beim thermischen Spritzen zu schützen, sollte auch die Betriebs- bzw. Prozessführung entsprechend angepasst werden. Soll beispielsweise eine maximale Temperatur von 120 °C im Bereich oder direkt an der Funktionskomponente 1 eingehalten werden, so kann dies über nachfolgende Spritzparameter und Kühlbedingungen beeinflusst werden.

Es erfolgt eine Senkung oder Steigerung der Pulverförderrate pro Spritzzyklus beim mehrfachen Überspritzen in mehreren einzelnen Schichten, bis eine geschlossene thermisch isolierende Schicht oder thermische Schicht ausgebildet worden ist. Beim Rotationsspritzverfahren wäre dies eine Überspritzung mit Schichtdicken im Bereich 10 µm - 50 µm. In der Regel sind drei Spritzübergänge für die Ausbildung einer geschlossenen Schicht erforderlich. Es kann so vorgegangen werden, dass zwei Überspritzungen mit geringerer Pulverförderrate durchgeführt werden. Danach wird eine Haltezeit eingehalten, bis Temperaturabsenkung auf eine Temperatur im Bereich 30 °C bis 40 °C erreicht worden ist. Nach Erreichen dieser Temperatur erfolgt eine weitere Überspritzung mit höherer Pulverförderrate, mit der dann die geschlossene Schicht ausgebildet worden ist. Danach erfolgt wieder eine Temperaturabsenkung auf eine Temperatur im Bereich 30 °C bis 40 °C, bevor eine weitere Schicht durch thermisches Spritzen oder ein weiterer Bearbeitungsschritt, bei dem es zu einer Erwärmung kommen kann, durchgeführt wird.

Es besteht die Möglichkeit, ein spannungssenkendes Anwärmen der Funktionskomponente 1 mit einer oder mehreren darauf ausgebildeten keramischen Schicht(en) vor einer nachfolgenden Überspritzung beispielsweise auf 50 °C durchzuführen. Der kurzfristig wirkende Temperaturgradient beim Überspritzen erreicht im Bereich der Funktionskomponente 1 eine Temperatur unterhalb von 60 °C bis 70 °C, d. h. eine Temperatur < 120 °C in Summe (Absoluttemperatur).

Ein inertes Schutzgas sollte orthogonal zur Spritzrichtung zugeführt werden und dabei einen mindestens ähnlich großen Bereich, den der Strahl beim thermischen Spritzen beeinflusst, überströmen. Der Schutzgasstrom kann in Abhängigkeit der auftretenden Temperaturen beeinflusst und bei Erwärmung dementsprechend der Volumenstrom erhöht werden. Schutzgas kann über mindestens eine Düse mit einem Druck im Bereich 5 bar bis 6 bar mit 40 bis 50 Normkubikmetern pro Stunde zugeführt werden. Vorteilhaft kann Schutzgas auch über zwei Düsen aus unterschiedlichen Richtungen zugeführt werden.

Ist neben dem Form- und Stoffschluss zwischen Funktionskomponente 1 und umgebendem Formkörper 2 auch ein Kraftschluss für eine Gradientenübertragung von Energie notwendig, sollte z. B. bei einem akustischen Wandler diese Oberfläche der Funktionskomponente 1 bis auf die Verbindungsschnittstelle nicht form- und stoffschlüssig mit dem Formkörper 2 verbunden sein. In diesem Falle würde auf die keramisch vergossene Baugruppe der Funktionskomponente 1 mittels thermischen Spritzens nach einer thermoisolierenden keramischen Oxidschicht 4 beispielsweise eine temperaturverteilende metallische Schicht 7 von 50 µm bis 200 µm Dicke gespritzt.

### Bezugszeichenliste

- 1: Funktionskomponente
- 2: Formkörper
- 2.1: Teilkörper (unterer, offen für Integration/Einbettung)
- 2.2: Teilkörper (oberer, abschließender nach Integration/Einbettung)
- 3: Trägerstruktur
- 3.1: Konturelement
- 3.2: Strahlschmelzzone
- 4: keramische Schicht
- 4.1: Längsnoppen
- 4.2: Einzelnoppe
- 4.3: Radialnoppe
- 5: Hohlraum
- 6: weitere keramische Schicht
- 7: metallische Schicht
- 8: Hülse
- 9.1: Oberteil
- 9.2: Mittelteil
- 9.3: Aussparung
- 9.4: Unterteil
- 10: Bereich
- 1 bis 14: stehen für die jeweilige Reihenfolge der Bestrahlung von Segmenten

## Patentansprüche

1. Verbundkörper mit mindestens einer Funktionskomponente (1) und einem Formkörper (2), wobei der generativ hergestellte Formkörper (2) die mindestens eine Funktionskomponente (1) ganz umschließt, **dadurch gekennzeichnet, dass**
- die mindestens eine Funktionskomponente (1) mit einer mindestens ein Konturelement (3.1) aufweisenden metallischen oder keramischen Trägerstruktur (3) in Verbindung stehend angeordnet ist,
- die mindestens eine Funktionskomponente (1) bis auf die Konturelemente (3.1) von mindestens einer thermisch isolierenden keramischen Schicht (4) umschlossen und darüber mindestens bereichsweise mit thermisch leitfähigem Metall oder Metalloxid beschichtet ist, so dass keine stoffschlüssige Verbindung zwischen Funktionskomponente (1) und Formkörper (2) besteht und
- der durch Strahlschmelzen eines Metallpulvers generativ hergestellte Formkörper (2) neben der mindestens einen Funktionskomponente (1) auch die Trägerstruktur (3) mit dem mindestens einen Konturelement (3.1) ganz umschließt, wobei von dem mindestens einen Konturelement (3.1) wenigstens eins eine stoffschlüssige Verbindung zum Formkörper (2) aufweist.

2. Verbundkörper nach Anspruch 1, **dadurch gekennzeichnet, dass** die Trägerstruktur (3) mindestens ein außen überstehendes Konturelement (3.1) aufweist, welches eingreifend in eine Strahlschmelzzone (3.2) des Formkörpers (2) angeordnet ist.

3. Verbundkörper nach Anspruch 1 oder 2, **dadurch gekennzeichnet, dass** die Trägerstruktur (3) zumindest bereichsweise, bevorzugt im Bereich der Konturelemente (3.1) mit einer metallischen Beschichtung versehen ist.

4. Verbundkörper nach einem der vorhergehenden Ansprüche, **dadurch gekennzeichnet, dass** die Funktionskomponente (1) ein Aktor und/oder ein Sensor und/oder ein Energiewandlerelement ist.

5. Verbundkörper nach einem der vorhergehenden Ansprüche, **dadurch gekennzeichnet, dass** die thermisch leitfähige Schicht eine höhere Schmelztemperatur als der Formkörper (2) aufweist.

6. Verbundkörper nach einem der Ansprüche 2 bis 5, **dadurch gekennzeichnet, dass** die Konturelemente (3.1) mindestens an den Bereichen, die an die Strahlschmelzzone (3.2) angrenzen, aus dem Metall des Formkörpers (2) oder einem Oxid des Werkstoffs, aus dem der Formkörper (2) gebildet ist, oder aus einem Metall oder einer Metalllegierung, das oder die mit dem Formkörperwerkstoff stoffschlüssig verbindbar ist, gebildet sind.

7. Verbundkörper nach Anspruch 6, **dadurch gekennzeichnet, dass** die Funktionskomponente (1) und/oder die Trägerstruktur (3) andere physikalische und/oder chemische Eigenschaften wie unterschiedliche Remanenz oder unterschiedliche Gitterstrukturen gegenüber dem Formkörper (2) aufweist.

8. Verbundkörper nach Anspruch 2, **dadurch gekennzeichnet, dass** in der Strahlschmelzzone (3.2) die Gefügestrukturen, wie Kristallform, Materialtyp oder Legierung, der Konturelemente (3.1) und der Formkörper (2) gleich sind.

9. Verbundkörper nach Anspruch 1, **dadurch gekennzeichnet, dass** die thermisch leitfähige Schicht eine Hülse (8) ist.

10. Verbundkörper nach Anspruch 9, **dadurch gekennzeichnet, dass** die Verbindung zwischen Hülse (8) und Formkörper (2) mindestens bereichsweise stoffschlüssig ist.

11. Verbundkörper nach einem der vorhergehenden Ansprüche, **dadurch gekennzeichnet, dass** die mindestens eine Funktionskomponente (1) form-, kraft- und/oder stoffschlüssig über thermisch isolierende Schichten mit der Trägerstruktur (3) verbunden ist.

12. Verbundkörper nach Anspruch 1, **dadurch gekennzeichnet, dass** ein Teilkörper (2.1, 9.2) vorhanden ist, in dem die Trägerstruktur (3) mit ihrer mindestens einen Funktionskomponente (1) angeordnet ist und über das mindestens eine Konturelement (3.1) stoffschlüssig verbunden ist, und der Teilkörper (2.1, 9.2) von einem weiteren Teilkörper (2.2, 9.1) gewölbeartig überbaut ist, wobei Teilkörper (2.1) und weiterer Teilkörper (2.2) stoffschlüssig verbunden sind.

13. Verbundkörper nach Anspruch 1, **dadurch gekennzeichnet, dass** zwischen äußerer Mantelfläche der mindestens einen Funktionskomponente (1) und angrenzender Innenwand des Formkörpers (2) Noppen (4.1, 4.2, 4.3) vorhanden sind, so dass ein nicht stoffschlüssig verschmolzener Raum mit thermisch isolierenden Eigenschaften ausgebildet ist.

14. Verfahren zur Herstellung eines Verbundkörpers, **dadurch gekennzeichnet, dass**
- mindestens eine Funktionskomponente (1) mit einer metallischen oder keramischen Trägerstruktur (3) mit mindestens einem Konturelement (3.1) verbunden wird,
- anschließend die mindestens eine Funktionskomponente (1) bis auf die Konturelemente (3.1) umschließend mit einer thermisch isolierenden keramischen Schicht (4) versehen wird, so dass im späteren Verlauf keine stoffschlüssige Verbindung zwischen Funktionskomponente (1) und einem Formkörper (2) entstehen kann,
- anschließend wird die thermisch isolierende Schicht (4) mindestens bereichsweise mit thermisch leitfähigem Metall oder Metalloxid beschichtet,
- anschließend ein Teilkörper (2.1, 9.2) mit mindestens einer Strahlschmelzzone (3.2) und Aussparung (9.3) zur Aufnahme der bisher hergestellten Einheit durch Strahlschmelzen eines Metallpulvers generativ und schichtweise hergestellt wird,
- anschließend die bisher hergestellte Einheit in den Teilkörper (2.1, 9.2) form-, kraft- und/oder stoffschlüssig in die Aussparung (9.3) eingesetzt wird, wobei die Trägerstruktur (3) mittelbar oder unmittelbar über Konturelemente (3.1) und/oder über Noppen (4.1, 4.2, 4.3) mit der Strahlschmelzzone (3.2) stoffschlüssig mit dem Teilkörper (2.1, 9.2) verbunden wird, und
- abschließend ebenfalls durch Strahlschmelzen eines Metallpulvers generativ und schichtweise die Aussparung (9.3) mit mindestens einem weiteren Teilkörper (2.2, 9.1, 9.4) überbaut wird, so dass ein monolithischer Formkörper (2) entsteht.

15. Verfahren nach Anspruch 14, **dadurch gekennzeichnet, dass** ein Energiestrahl, insbesondere ein Laser- oder Elektronenstrahl, beim Strahlschmelzen so betrieben und mit seinem Brennfleck so bewegt wird, dass eine für den Funktionserhalt der jeweiligen Funktionskomponente (1) spezifische Maximaltemperatur während der Herstellung des Formkörpers (2) nicht überschritten wird.

16. Verfahren nach Anspruch 15, **dadurch gekennzeichnet, dass** die Energiedichte im Brennfleck des Energiestrahls, die Vorschubgeschwindigkeit des Brennflecks, der Abstand von Schmelzspuren, die jeweilige Schichtdicke der Pulverschichten und/oder das Bestrahlungsmuster so beeinflusst werden, dass die spezifische Maximaltemperatur an der Funktionskomponente (1) nicht erreicht wird.

17. Verfahren nach Anspruch 14 bis 16, **dadurch gekennzeichnet, dass** das aufgetragene Metallpulver und/oder die darunter liegenden bereits geschmolzenen Schichten vor dem Strahlschmelzen der aufgetragenen Metallpulverschicht vorgewärmt werden.

18. Verfahren nach einem der Ansprüche 14 bis 16, **dadurch gekennzeichnet, dass** eine gefüge- und funktionserhaltende maximale Reduktion des Energieeintrages in der Nähe temperaturempfindlicher Teile der Funktionskomponente (1) erfolgt und mit zunehmendem Abstand dazu die Reduktion des Energieeintrags abnimmt.

19. Verfahren nach Anspruch 14, **dadurch gekennzeichnet, dass** der Formkörper (2) aus mehreren Teilkörpern (2.1, 2.2 bzw. 9.2, 9.1, 9.4), die generativ durch Strahlschmelzen hergestellt und stoffschlüssig, insbesondere beim Strahlschmelzen, miteinander verbunden werden, hergestellt wird.

20. Verfahren nach Anspruch 14, **dadurch gekennzeichnet, dass** die thermisch isolierende Schicht und/oder die thermisch leitfähige Schicht mit mehreren übereinander ausgebildeten Einzelschichten durch thermisches Spritzen ausgebildet wird und dabei Haltephasen zum Abkühlen eingehalten werden und/oder das thermische Spritzen unter Vakuumbedingungen oder unter Einsatz eines inerten Schutzgases, mit dem bevorzugt eine Kühlung erreichbar ist, durchgeführt wird.

21. Verfahren nach Anspruch 14, **dadurch gekennzeichnet, dass** der Formkörper (2) mindestens teilweise ausgehend von den Konturelementen (3.1) mittels Strahlschmelzen hergestellt wird.

22. Verfahren nach Anspruch 14, **dadurch gekennzeichnet, dass** zwischen Außenkontur der mindestens einen beschichteten Funktionskomponente (1) und Formkörper (2) ein nicht stoffschlüssig verschmolzener Raum ausgebildet wird, der thermisch isolierende Eigenschaften aufweist.

23. Verfahren nach Anspruch 15, **dadurch gekennzeichnet, dass** der aufzuschmelzende Formkörper (2) bzw. eine darunter befindliche Bauplattform und/oder die Zuführung des Metallpulvers und/oder die Führung des Energiestrahls in 3-D beweglich ausgeführt wird.

24. Verfahren nach einem der Ansprüche 14 bis 23, **dadurch gekennzeichnet, dass** eine Überwachung und/oder Steuerung des Volumenenergieeintrages und/oder eine Regelung des Strahlschmelzprozesses mit zwei Messverfahren, die unterschiedliche physikalische, sich gegenseitig nicht beeinflussende Messprinzipien, vorzugsweise optisch und akustisch, realisieren, durchgeführt werden.

## Claims

1. A composite body comprising at least one functional component (1) and a moulded body (2), wherein the generatively produced moulded body (2) completely encloses the at least one functional component (1), **characterised in that**
- the at least one functional component (1) is arranged in connection with a metallic or ceramic carrier structure (3) having at least one contour element (3.1),
- the at least one functional component (1), except for the contour elements (3.1), is enclosed by at least one thermally insulating ceramic layer (4), above which it is coated at least in some areas with thermally conductive metal or metal oxide, so that there is no cohesive bond between the functional component (1) and the moulded body (2), and
- the moulded body (2) produced generatively by beam melting of a metal powder, in addition to the at least one functional component (1), also completely encloses the carrier structure (3) with the at least one contour element (3.1), at least one of the at least one contour element (3.1) having a cohesive bond with the moulded body (2).

2. The composite body according to claim 1, **characterised in that** the carrier structure (3) has at least one externally projecting contour element (3.1) which is arranged to engage in a beam melting zone (3.2) of the moulded body (2).

3. The composite body according to claim 1 or 2, **characterised in that** the carrier structure (3) is provided with a metallic coating at least in some regions, preferably in the region of the contour elements (3.1).

4. The composite body according to any one of the preceding claims, **characterised in that** the functional component (1) is an actuator and/or a sensor and/or an energy conversion element.

5. The composite body according to any one of the preceding claims, **characterised in that** the thermally conductive layer has a higher melting temperature than the moulded body (2).

6. The composite body according to any one of claims 2 to 5, **characterised in that** the contour elements (3.1), at least at the regions which adjoin the beam melting zone (3.2), are formed from the metal of the moulded body (2) or an oxide of the material from which the moulded body (2) is formed, or from a metal or a metal alloy which can be cohesively bonded to the moulded body material.

7. The composite body according to claim 6, **characterised in that** the functional component (1) and/or the carrier structure (3) has different physical and/or chemical properties such as different remanence or different lattice structures with respect to the moulded body (2).

8. The composite body according to claim 2, **characterised in that** in the beam melting zone (3.2) the microstructures, such as crystal form, material type or alloy, of the contour elements (3.1) and the moulded body (2) are identical.

9. The composite body according to claim 1, **characterised in that** the thermally conductive layer is a sleeve (8).

10. The composite body according to claim 9, **characterised in that** the connection between the sleeve (8) and the moulded body (2) is a cohesive bond at least in some regions.

11. The composite body according to any one of the preceding claims, **characterised in that** the at least one functional component (1) is connected to the carrier structure (3) in a form-fitting, force-fitting and/or cohesively bonded manner via thermally insulating layers.

12. The composite body according to claim 1, **characterised in that** a partial body (2.1, 9.2) is present in which the carrier structure (3) with its at least one functional component (1) is arranged and is cohesively bonded via the at least one contour element (3.1), and the partial body (2.1, 9.2) is covered in the manner of a vault by a further partial body (2.2, 9.1), the partial body (2.1) and the further partial body (2.2) being cohesively bonded.

13. The composite body according to claim 1, **characterised in that** nubs (4.1, 4.2, 4.3) are present between the outer shell of the at least one functional component (1) and the adjacent inner wall of the moulded body (2), so that a space with thermally insulating properties is formed which is not fused in a cohesively bonding manner.

14. A method for producing a composite body, **characterised in that**
- at least one functional component (1) is connected with a metallic or ceramic carrier structure (3) having at least one contour element (3.1),
- then the at least one functional component (1), except for the contour elements (3.1), is provided in an enclosing manner with at least one thermally insulating ceramic layer (4), so that no cohesive bond can subsequently form between the functional component (1) and the moulded body (2), and
- then the thermally insulating layer (4) is coated at least in some regions with thermally conductive metal or metal oxide,
- then a partial body (2.1, 9.2) with at least one beam melting zone (3.2) and recess (9.3) for receiving the previously produced unit is produced generatively and layerwise by beam melting of a metal powder,
- then the previously produced unit is inserted into the partial body (2.1, 9.2) in the recess (9.3) in a form-fitting, force-fitting and/or cohesively bonded manner, the carrier structure (3) being connected directly or indirectly via contour elements (3.1) and/or via nubs (4.1, 4.2, 4.3) to the beam melting zone (3.2) in a cohesively bonded manner to the partial body (2.1, 9.2), and
- finally, the recess (9.3) is also generatively and layerwise covered with at least one further partial body (2.2, 9.1, 9.4) by beam melting of a metal powder, so that a monolithic moulded body (2) is produced.

15. The method according to claim 14, **characterised in that** an energy beam, in particular a laser or electron beam, is operated during beam melting and moved with its focal spot in such a way that a maximum temperature specific to the functional maintenance of the respective functional component (1) is not exceeded during the production of the moulded body (2).

16. The method according to claim 15, **characterised in that** the energy density in the focal spot of the energy beam, the feed rate of the focal spot, the distance between melt tracks, the respective layer thickness of the powder layers and/or the irradiation pattern are influenced such that the specific maximum temperature is not reached at the functional component (1).

17. The method according to claims 14 to 16, **characterised in that** the applied metal powder and/or the already molten layers underneath are preheated before the beam melting of the applied metal powder layer.

18. The method according to any one of claims 14 to 16, **characterised in that** a structure- and function-preserving maximum reduction of the energy input takes place in the vicinity of temperature-sensitive parts of the functional component (1) and with increasing distance therefrom the reduction of the energy input decreases.

19. The method according to claim 14, **characterised in that** the moulded body (2) is produced from a plurality of partial bodies (2.1, 2.2 or 9.2, 9.1, 9.4, respectively), which are produced generatively by beam melting and are connected to one another by a cohesive bond, in particular during beam melting.

20. The method according to claim 14, **characterised in that** the thermally insulating layer and/or the thermally conductive layer is formed with several individual layers formed one above the other by thermal spraying and holding phases for cooling are observed and/or the thermal spraying is carried out under vacuum conditions or using an inert protective gas with which cooling can preferably be achieved.

21. The method according to claim 14, **characterised in that** the moulded body (2) is produced at least partially starting from the contour elements (3.1) by means of beam melting.

22. The method according to claim 14, **characterised in that** between the outer contour of the at least one coated functional component (1) and the moulded body (2) there is formed a space which is not fused in a cohesively bonding manner and has thermal insulation properties.

23. The method according to claim 15, **characterised in that** the moulded body (2) to be melted or a building platform located below it and/or the supply of the metal powder and/or the guidance of the energy beam is carried out movably in 3D.

24. The method according to any one of claims 14 to 23, **characterised in that** monitoring and/or control of the volumetric energy input and/or control of the beam melting process is carried out with two measuring methods which implement different physical measuring principles which do not influence each other, preferably optical and acoustic principles.

## Revendications

1. Corps composite comprenant au moins un élément fonctionnel (1) et un corps façonné (2), le corps façonné (2) fabriqué par génération entourant complètement ledit au moins un élément fonctionnel (1), **caractérisé en ce que**
- ledit au moins un élément fonctionnel (1) est disposé en liaison avec une structure de support métallique ou céramique (3) comportant au moins un élément de contour (3.1),
- ledit au moins un élément fonctionnel (1) est entouré, à l'exception des éléments de contour (3.1), d'au moins une couche céramique (4) thermiquement isolante et est revêtu par-dessus, au moins par endroits, d'un métal ou d'un oxyde métallique thermiquement conducteur, de sorte qu'il n'existe aucune liaison de matière entre l'élément fonctionnel (1) et le corps façonné (2), et
- le corps façonné (2) fabriqué de manière générative par fusion par faisceau d'une poudre métallique, en plus dudit au moins un élément fonctionnel (1), entoure également complètement la structure de support (3) avec ledit au moins un élément de contour (3.1), au moins un des au moins un élément de contour (3.1) ayant une liaison de matière avec le corps façonné (2).

2. Corps composite selon la revendication 1, **caractérisé en ce que** la structure de support (3) comporte au moins un élément de contour (3.1) faisant saillie vers l'extérieur, qui est agencé pour s'engager dans une zone de fusion par faisceau (3.2) du corps façonné (2).

3. Corps composite selon la revendication 1 ou 2, **caractérisé en ce que** la structure de support (3) est pourvue d'un revêtement métallique au moins dans certaines régions, de préférence dans la région des éléments de contour (3.1).

4. Corps composite selon l'une quelconque des revendications précédentes, **caractérisé en ce que** l'élément fonctionnel (1) est un actionneur et/ou un capteur et/ou un élément de conversion d'énergie.

5. Corps composite selon l'une quelconque des revendications précédentes, **caractérisé en ce que** la couche thermiquement conductrice a une température de fusion plus élevée que le corps façonné (2).

6. Corps composite selon l'une quelconque des revendications 2 à 5, **caractérisé en ce que** les éléments de contour (3.1), au moins dans les régions adjacentes à la zone de fusion par faisceau (3.2), sont formés à partir du métal du corps façonné (2) ou d'un oxyde du matériau à partir duquel le corps façonné (2) est formé, ou à partir d'un métal ou d'un alliage métallique qui peut être lié au matériau du corps façonné par liaison de matière.

7. Corps composite selon la revendication 6, **caractérisé en ce que** l'élément fonctionnel (1) et/ou la structure de support (3) présente des propriétés physiques et/ou chimiques différentes, telles qu'une rémanence différente ou des structures réticulaires différentes par rapport au corps façonné (2).

8. Corps composite selon la revendication 2, **caractérisé en ce que** dans la zone de fusion par faisceau (3.2), les microstructures, telles que la forme cristalline, le type de matériau ou l'alliage, des éléments de contour (3.1) et le corps façonné (2) sont identiques.

9. Corps composite selon la revendication 1, **caractérisé en ce que** la couche thermiquement conductrice est une gaine (8).

10. Corps composite selon la revendication 9, **caractérisé en ce que** la liaison entre la gaine (8) et le corps façonné (2) est une liaison de matière au moins dans certaines régions.

11. Corps composite selon l'une quelconque des revendications précédentes, **caractérisé en ce que** l'au moins un élément fonctionnel (1) est relié à la structure de support (3) par des couches thermiquement isolatrices par liaison de forme, de force et/ou de matière.

12. Corps composite selon la revendication 1, **caractérisé en ce qu'**il existe un corps partiel (2.1, 9.2) dans lequel est disposée la structure de support (3) avec son au moins un élément fonctionnel (1) et qui est relié par liaison de matière par l'intermédiaire dudit au moins un élément de contour (3.1), et le corps partiel (2.1, 9.2) est recouvert à la manière d'une voûte par un autre corps partiel (2.2, 9.1), le corps partiel (2.1) et l'autre corps partiel (2.2) étant reliés par liaison de matière.

13. Corps composite selon la revendication 1, **caractérisé en ce que** des nopes (4.1, 4.2, 4.3) sont présentes entre la surface circonférentielle extérieure dudit au moins un élément fonctionnel (1) et la paroi intérieure adjacente du corps façonné (2), de sorte qu'il se forme un espace qui n'est pas fusionné de manière à établir une liaison de matière et qui présente des propriétés d'isolation thermique.

14. Procédé de fabrication d'un corps composite, **caractérisé en ce que**
- au moins un élément fonctionnel (1) est relié à une structure de support métallique ou céramique (3) avec au moins un élément de contour (3.1),
- alors ledit au moins un élément fonctionnel (1), à l'exception des éléments de contour (3.1), est pourvu, de manière entourante, d'une couche céramique (4) thermiquement isolante, de sorte qu'aucune liaison de matière entre l'élément fonctionnel (1) et un corps façonné (2) ne peut être formée au cours du processus ultérieur,
- la couche thermiquement isolante (4) est alors recouverte, au moins par endroits, d'un métal ou d'un oxyde métallique thermiquement conducteur,
- puis un corps partiel (2.1, 9.2) avec au moins une zone de fusion par faisceau (3.2) et un évidement (9.3) pour recevoir l'ensemble produit précédemment est produit de manière générative et par couches au moyen d'une fusion par faisceau d'une poudre métallique,
- puis l'ensemble précédemment fabriqué est inséré dans le corps partiel (2.1, 9.2) dans l'évidement (9.3) par liaison de forme, de force et/ou de matière, la structure de support (3) étant reliée directement ou indirectement par des éléments de contour (3.1) et/ou par des nopes (4.1, 4.2, 4.3) à la zone de fusion par faisceau (3.2) par liaison de matière avec le corps partiel (2.1, 9.2), et
- enfin, également au moyen d'une fusion par faisceau d'une poudre métallique, l'évidement (9.3) est recouvert de manière générative et par couches d'au moins un autre corps partiel (2.2, 9.1, 9.4), de sorte qu'un corps façonné monolithique (2) est produit.

15. Procédé selon la revendication 14, **caractérisé en ce qu'**un faisceau d'énergie, notamment un faisceau laser ou un faisceau d'électrons, est utilisé pendant la fusion par faisceau et est déplacé avec son point focal de telle sorte qu'une température maximale spécifique pour le maintien fonctionnel de l'élément fonctionnel respectif (1) ne soit pas dépassée pendant la fabrication du corps façonné (2).

16. Procédé selon la revendication 15, **caractérisé en ce que** la densité d'énergie dans le point focal du faisceau d'énergie, la vitesse d'avance du point focal, la distance des pistes de fusion, l'épaisseur respective des couches de poudre et/ou le schéma d'irradiation sont influencés de telle manière que la température maximale spécifique n'est pas atteinte au niveau de l'élément fonctionnel (1).

17. Procédé selon les revendications 14 à 16, **caractérisé en ce que** la poudre métallique appliquée et/ou les couches déjà fondues en dessous sont préchauffées avant la fusion par faisceau de la couche de poudre métallique appliquée.

18. Procédé selon l'une quelconque des revendications 14 à 16, **caractérisé en ce qu'**une réduction maximale de l'apport d'énergie préservant la structure et la fonction a lieu à proximité des parties sensibles à la température de l'élément fonctionnel (1) et la réduction de l'apport d'énergie diminue à mesure que l'on s'éloigne de celles-ci.

19. Procédé selon la revendication 14, **caractérisé en ce que** le corps façonné (2) est fabriqué à partir de plusieurs corps partiels (2.1, 2.2 ou 9.2, 9.1, 9.4), qui sont fabriqués de manière générative au moyen d'une fusion par faisceau et qui sont reliés entre eux par liaison de matière, notamment lors de la fusion par faisceau.

20. Procédé selon la revendication 14, **caractérisé en ce que** la couche thermiquement isolante et/ou la couche thermiquement conductrice est formée de plusieurs couches individuelles formées les unes au-dessus des autres par projection thermique et des phases de maintien pour le refroidissement sont maintenues et/ou la projection thermique est effectuée dans des conditions de vide ou en utilisant un gaz protecteur inerte permettant d'obtenir de préférence un refroidissement.

21. Procédé selon la revendication 14, **caractérisé en ce que** le corps façonné (2) est produit au moins partiellement à partir des éléments de contour (3.1) au moyen d'une fusion par faisceau.

22. Procédé selon la revendication 14, **caractérisé en ce qu'**un espace non fondu de manière à établir une liasion de matière est formé entre le contour extérieur d'au moins un élément fonctionnel revêtu (1) et le corps façonné (2), lequel espace présente des propriétés d'isolation thermique.

23. Procédé selon la revendication 15, **caractérisé en ce que** le corps façonné (2) à fondre ou une plate-forme de construction située en dessous et/ou l'alimentation de la poudre métallique et/ou le guidage du faisceau d'énergie sont effectués de manière mobile en 3D.

24. Procédé selon l'une quelconque des revendications 14 à 23, **caractérisé en ce que** la surveillance et/ou le contrôle de l'apport d'énergie volumique et/ou la régulation du processus de fusion par faisceau sont effectués avec deux méthodes de mesure qui mettent en œuvre des principes de mesure physiques différents qui ne s'influencent pas mutuellement, de préférence des principes optiques et acoustiques.
